(19) Europäisches Patentamt
European Patent Office
Office européen des brevets

(11) EP 0 946 744 B1

(12) FASCICULE DE BREVET EUROPEEN

(45) Date de publication et mention de la délivrance du brevet:
**18.08.2004 Bulletin 2004/34**

(21) Numéro de dépôt: **97945903.9**

(22) Date de dépôt: **10.11.1997**

(51) Int Cl.⁷: **C12N 15/88**, A61K 48/00

(86) Numéro de dépôt international:
**PCT/FR1997/002022**

(87) Numéro de publication internationale:
**WO 1998/022610 (28.05.1998 Gazette 1998/21)**

# (54) NOUVEAUX COMPLEXES POLYMERIQUES POUR LA TRANSFECTION D'ACIDES NUCLEIQUES, AVEC DES RESIDUS DESTABILISANT DES MEMBRANES CELLULAIRES

NEUE POLYMERISCHE KOMPLEXE FÜR DIE TRANSFEKTION VON NUKLEINSAÜREN, MIT RESTEN FÜR DESTABILISIERUNG DER ZELLMEMBRANEN

NOVEL POLYMERIC COMPLEXES FOR THE TRANSFECTION OF NUCLEIC ACIDS, WITH RESIDUES CAUSING THE DESTABILISATION OF CELL MEMBRANES

(84) Etats contractants désignés:
**AT BE CH DE DK ES FI FR GB GR IE IT LI LU MC NL PT SE**

(30) Priorité: **15.11.1996 FR 9613990**

(43) Date de publication de la demande:
**06.10.1999 Bulletin 1999/40**

(73) Titulaire: **I.D.M. IMMUNO-DESIGNED MOLECULES**
**75011 Paris (FR)**

(72) Inventeurs:
- **MIDOUX, Patrick**
**F-45100 Orléans (FR)**
- **MONSIGNY, Michel**
**F-45590 Saint-Cyr-en-Val (FR)**

(74) Mandataire: **Grosset-Fournier, Chantal Catherine**
**Grosset-Fournier & Demachy,**
**54, rue Saint-Lazare**
**75009 Paris (FR)**

(56) Documents cités:

| | |
|---|---|
| **EP-A- 0 350 246** | **EP-A- 0 387 775** |
| **EP-A- 0 388 758** | **WO-A-92/11037** |
| **WO-A-92/13570** | **FR-A- 2 107 756** |
| **FR-A- 2 719 316** | **US-A- 5 166 320** |

- **MEZÖ G. ET AL.: "Carrier design:conformational studies of amino acid (X) and oligopeptide (X-DL-Ala-m) substituted poly(L-lysine)." BIOPOLYMERS, vol. 33, no. 6, 1993, pages 873-885, XP002034603**
- **WANG C. Y. ET AL: "POLYHISTIDINE MEDIATES AN ACID-DEPENDENT FUSION OF NEGATIVELY CHARGED LIPOSOMES" BIOCHEMISTRY, vol. 23, no. 19, 1984, pages 4409-4416, XP002016041 cité dans la demande**
- **MIDOUX P ET AL: "SPECIFIC GENE TRANSFER MEDIATED BY LACTOSYLATED POLY-L-LYSINE INTO HEPATOMA CELLS" NUCLEIC ACIDS RESEARCH, vol. 21, no. 4, 25 février 1993, pages 871-878, XP000371764 cité dans la demande**
- **SANTELLA M. AND LI H.J.: 'Interaction between poly(L-Lysine, L-Histidine) and DNA' BIOPOLYMERS vol. 16, 1997, pages 1879 - 1894**
- **ROUFAI M.B. AND MIDOUX P.: 'Histidylated polylysine as DNA vector: Elevation of the imidazole protonation and reduced cellular uptake without change in the polyfection efficiency of serum stabilized negative polyplexes' BIOCONJUGATE CHEMISTRY vol. 12, no. 1, pages 92 - 99**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

L'invention a pour objet de nouveaux complexes d'acides nucléiques et de polymère substitué par des résidus entraînant la déstabilisation des membranes cellulaires.

L'introduction d'un gène étranger dans une cellule est la base de la thérapie génique. Le transfert de gènes peut être obtenu en utilisant soit un matériel viral modifié (virus de la vaccine, rétrovirus, adénovirus ou virus de l'herpès), soit en utilisant des vecteurs non viraux (lipides cationiques, liposomes). Les premiers bien qu'efficaces, présentent des problèmes de sécurité. Pour les derniers, l'efficacité est fortement diminuée en présence de sérum et de ce fait leur utilisation est restreinte à l'*in vitro* ou à l'*ex vivo*.

La polylysine qui peut former des complexes électrostatiques stables avec un ADN plasmidique est à la base du développement de vecteurs non viraux pour transférer des gènes dans des cellules animales.

Les complexes d'ADN et de polylysine non substituée ne sont généralement pas efficaces pour transfecter des cellules du fait de la très grande stabilité des complexes (donc dissociation et relargage de l'ADN faibles) dans les conditions physiologiques par suite d'une très grande coopérativité des interactions polycation-polyanion.

L'efficacité de transfection peut être améliorée lorsque le nombre de charges présent sur le polypeptide est diminué afin de réduire les forces d'interactions entre l'ADN et la polylysine. Par exemple, lorsque 40% des fonctions $\varepsilon$-$NH_3^+$ des résidus lysine de la polylysine sont partiellement neutralisées par des dérivés polyhydroxyalcanoyles tels que la $\delta$-gluconolactone, les complexes ADN/polylysine partiellement gluconoylée sont plus efficaces que les complexes ADN/polylysine pour transfecter les cellules.

La polylysine peut être substituée par des ligands spécifiques de récepteurs présents à la surface des cellules et capables d'induire une endocytose spécifique des complexes avec un ADN plasmidique par des cellules cibles.

Des conjugués obtenus en substituant de la polylysine par l'asialoorosomucoïde, la transferrine, l'insuline, des immunoglobulines et des facteurs de croissance ont été proposés comme vecteurs guides de plasmides. Cependant, ces ligands protéiques rendent les complexes très immunogéniques.

La polylysine peut être substituée par des ligands de petite masse moléculaire moins immunogènes tels que des osides et oligosides reconnus par des récepteurs membranaires spécifiques (lectines membranaires) à la surface des cellules cibles. La polylysine glycosylée a été proposée comme vecteurs non viraux parfaitement définis pour transférer des gènes.

De nombreuses cellules animales possèdent des lectines membranaires qui reconnaissent des oligosides de structures variées et qui induisent l'endocytose de leurs ligands. Par exemple, la lectine membranaire des cellules du parenchyme hépatique reconnaît des structures glucidiques comportant un résidu galactose en position terminale, ce qui est le cas des glycoprotéines sériques désialylées. La spécificité des lectines membranaires dépend du type cellulaire et de l'état de différenciation des cellules.

L'efficacité de la transfection des complexes ADN/polylysine glycosylée dépend du taux de substitution de la polylysine par des osides : les transfections les plus efficaces sont obtenues lorsque 30 à 40% des fonctions $\varepsilon$-$NH_3^+$ des résidus lysine de la polylysine sont substituées par des mono- ou des dissacharides.

Dans le brevet français n° 2,719,316, il a été montré que l'utilisation de la polylysine partiellement gluconoylée comportant un nombre de charges positives déjà réduit permet d'abaisser d'un facteur 5 à 10 le nombre d'osides qu'il est nécessaire de fixer sur le polymère pour obtenir une bonne efficacité de transfection des complexes ADN/polylysine glycosylée et gluconoylée. L'utilisation de la polylysine partiellement gluconoylée permet d'augmenter la solubilité des complexes et de réduire leur taille aux environs de 50 nm.

Le transport: des plasmides par des vecteurs non viraux susceptibles d'être reconnus spécifiquement par des composés de la membrane plasmique des cellules relève d'une démarche imitant le mécanisme d'entrée du matériel génétique viral dans une cellule. Dans tous les cas décrits, les complexes ADN/polymère polycationique sont entraînés dans des vésicules d'endocytose, dans des endosomes et probablement dans d'autres compartiments intracellulaires plus profonds, éloignés de la membrane plasmique.

Le passage transmembranaire de l'ADN plasmidique est de ce fait une étape critique vis à vis de la libération dudit ADN dans le cytosol pour son passage dans le noyau, où le gène sera exprimé.

Dans tous les cas décrits, des auxiliaires de passage transmembranaire sont utilisés pour favoriser le passage de l'ADN dans le cytosol. Il s'agit :

- de la chloroquine
- d'adénovirus défectifs
- des peptides perméabilisants et/ou fusiogènes

a) La chloroquine est une base faible utilisée à 50 μM ou 100 μM en culture *in vitro*, pour certaines cellules ces concentrations sont toxiques. La chloroquine, qui est perméante traverse la membrane et s'accumule dans les compartiments acides, parce qu'elle comporte des amines de faible pK qui captent des protons ; la

chloroquine protonée est cationique et moins perméante. L'acidification des endosomes et des lysosomes est due à une enzyme membranaire qui injecte des $H^+$ à partir du cytosol, dans les vésicules ; pour rétablir l'électroneutralité cette accumulation de proton s'accompagne d'une entrée d'ions chlorure Cl-. Au fur et à mesure que la chloroquine s'accumule, les protons et les chlorures s'accumulent également, ce qui provoque une augmentation de la force ionique intravésiculaire qui induit l'arrivée d'eau, d'où le gonflement des vésicules et leur déstabilisation. La concentration intracellulaire de la chloroquine peut être plus de 100 fois supérieure à sa concentration dans le milieu, après quelques heures. Elle peut donc dépasser 10 mM. Ce phénomène est comparable à ce qui se passe chez les personnes qui utilisent une dose quotidienne de 300 mg de chloroquine par jour. Après quelques jours, la concentration plasmatique est aux environs de 0,7 µM, la concentration tissulaire est 200 à 700 fois plus élevée, soit 140 à 500 µM. Et à l'intérieur des cellules, les compartiments acides peuvent atteindre des concentrations plusieurs dizaines de fois plus fortes. On sait par ailleurs que des concentrations 10 mM de chloroquine (concentration obtenue après quelques heures en ayant utilisé une concentration initiale de chloroquine de 100 µM) favorise la dissociation des complexes ADN/polylysine.

La chloroquine en association avec les complexes ADN/polylysine dans le transfert de gène n'est utilisable que dans des applications *in vitro* ou *ex vivo* du fait de sa toxicité et de sa rapide dilution après injection chez l'individu. En effet, *in vivo*, pour atteindre les concentrations élevées citées ci-dessus, il faut plusieurs jours. Or il a été montré *in vitro* que dans les cellules prétraitées à la chloroquine, l'expression des gènes transférés était très faible. En outre, si la chloroquine est ajoutée plus de trois heures après l'incubation des cellules en présence des complexes, la transfection est très faible. C'est pour ces raisons que la chloroquine, qui est un très bon auxiliaire *in vitro*, n'est pas efficace *in vivo*.

b) Les propriétés fusiogènes en milieu acide des particules d'adénovirus défectifs sont utilisées pour favoriser le passage de l'ADN dans le cytosol à partir des vésicules d'endocytose. Les adénovirus possèdent des protéines de fusion actives en milieu légèrement acide. Les adénovirus défectifs peuvent être utilisés libres ou fixés aux complexes ADN/polylysine.

L'utilisation de particules virales même défectives pose cependant des problèmes de sécurité. Les adénovirus induisent une très forte réponse immune après injection avec les complexes.

c) Des peptides perméabilisants et/ou fusiogènes en milieu légèrement acide sont utilisés comme auxiliaires pour favoriser le passage de l'ADN dans le cytosol. Il s'agit principalement de peptides de 20 acides aminés dérivant des protéines de fusion de virus comme par exemple le peptide *N*-terminal de la sous unité HA2 de l'hémagglutinine du virus de la grippe, ou de peptides synthétiques tels que le GALA, un oligomère contenant plusieurs unités de répétition Glu-Ala-Leu-Ala. Ces peptides sont utilisés le plus souvent à l'état libre (c'est-à-dire non fixés covalemment) avec les complexes ADN/polylysine. L'efficacité des peptides est fortement diminuée en présence de sérum dans les milieu de culture cellulaire ce qui restreint leur utilisation aux expériences *in vitro* ou au *ex vivo*. Certains peptides fixés covalemment aux complexes ADN/polylysine sont encore efficaces pour favoriser le passage transmembranaire de l'ADN, d'autres perdent leur pouvoir perméabilisant après fixation.

On sait, par ailleurs, qu'il existe d'autres molécules capables de déstabiliser des membranes et en particulier des molécules contenant le noyau imidazole de l'histidine (pK = 6,04) qui en se protonant en milieu légèrement acide devient cationique. La polyhistidine possède des propriétés fusiogènes et perméabilisantes vis-à-vis des bicouches lipidiques. A pH <6, la polyhistidine adopte une structure en hélice $\alpha$ (Norland K.S. *et al*. (1963) Biopolymers 1:277-278 ; Beychok S. *et al*. (1965) J. Amer. Chem. Soc. 87:3990-3991). Il a été montré que la polyhistidine en milieu légèrement acide est un polycation qui agrège des liposomes chargés négativement et induit leur fusion (Wang C.-Y. et Huang L. (1984) Polyhistidine mediates an acid-dependent fusion of negatively charged liposomes. Biochemistry 23:4409-4416 ; Uster P.S. et Deamer D.W. (1985) pH-dependent fusion of liposomes using titrable polycations. Biochemistry 24:8-14).

On sait qu'un polymère synthétique (la cétylacétyl(imidazol-4-ylméthyl)polyéthyleimine) à pH légèrement acide induit la fusion de liposomes (Oku N. *et al*. (1987) Low pH induced membrane fusion of lipid vesicles containing proton-sensitive polymer. Biochemistry 26:8145-8150).

On sait également qu'un polymère neutre hydrosoluble substitué par des résidus histidyle (utilisé à la place de la polyhistidine très peu soluble en milieu aqueux) interagit uniquement en milieu légèrement acide avec un polyanion tel que l'acide polyaspartique et est capable de perméabiliser la membrane plasmique des cellules dans un test de cytométrie en flux en utilisant le bromure d'éthidium comme marqueur (Midoux *et al*., 1995).

Des résultats préliminaires ont montré que la polyhistidine (très peu soluble en milieu aqueux à pH neutre) ne peut être utilisée pour transfecter des cellules car n' étant pas un polycation à pH neutre, elle n'est pas capable de former avec de l'ADN des complexes stables ayant une solubilité suffisante pour pouvoir être utilisé à pH neutre, en particulier à pH 7,4, le pH du plasma.

La demande de brevet européen EP/0941/123, publiée sous le numéro WO98/19710 le 14 mai 1998 revendiquant la priorité GB/97 02 965 du 6 novembre 1996, n'est à prendre en considération qu'au titre de la nouveauté.

Elle divulgue la formation d'un complexe d'ADN et d'une polyamine modifiée, plus particulièrement une polylysine, sur laquelle est greffée une molécule de polyéthylèneglycol par l'intermédiaire d'une liaison disulfure introduite par réaction à l'acide N-succinimidyl 3-(2-pyridyldithio)propionate. Ce document décrit également l'utilisation de ces complexes en thérapies.

Cependant, ces complexes utilisés pour permettre le transfert d'ADN dans des cellules cibles. ne contiennent pas de résidus entraînant la déstabilisation de membranes cellulaires en milieu faiblement acide.

Le document intitulé Carrier design Biopolymers, vol. 33, 873-885 (1993) Mezö et al. concerne des études de conformation sur de la poly(L-lysine), substituée par des aminoacides et des oligopeptides.

Cependant, ce document ne traite pas des problèmes de déstabilisation des membranes cellulaires en milieu faiblement acide, pour permettre le transfert d'ADN dans des cellules cibles.

Les nouveaux complexes d'acide nucléique et de polymère substitué sont susceptibles de transfecter plusieurs types cellulaires.

Un nouveau type de polymère cationique comprend, outre les charges positives du polymère, des substituants favorisant le passage transmembranaire de l'acide nucléique transporté et, le cas échéant,des substituants agissant en tant que signaux de reconnaissance. Les substituants favorisant le passage transmembranaire sont liés au polymère et sont des dérivés qui ne sont pas cationiques en milieu légèrement alcalin, mais le deviennent en milieu neutre et en milieu acide.

Les nouveaux complexes d'acide nucléique et de polymère substitué susceptibles de favoriser le passage transmembranaire de l'ADN après endocytose des complexes.

Les nouveaux complexes d'acide nucléique et de polymère substitué ne présentent pas de signaux de reconnaissance reconnus par des récepteurs membranaires à la surface des cellules.

Les nouveaux complexes d'acide nucléique et de polymère substitué présentent en outre des signaux de reconnaissance reconnus par des récepteurs membranaires à la surface des cellules, conférant un caractère sélectif de la transfection vis-à-vis de différents types cellulaires.

Un procédé de transfection spécifique *in vitro* et *in vivo* est décrit.

Les nouveaux conjugués de polylysine substituée ne présentent pas de signaux de reconnaissance reconnus par des récepteurs membranaires à la surface des cellules, susceptibles d'être complexés à un acide nucléique en vue de la transfection d'une cellule.

Les nouveaux conjugués de polylysine présentent en outre des signaux de reconnaissance reconnus par des récepteurs membranaires à la surface des cellules, susceptibles d'être complexés à un acide nucléique en vue de la transfection sélective d'une cellule.

L'intérêtde l'invention est que ces nouveaux complexes d'acide nucléique et de polymère sont susceptibles de transfecter les cellules en l'absence d'auxiliaires de passage transmembranaire (chloroquine ou peptides perméabilisants et/ou fusiogènes). Ce sont ici les groupements faiblement basiques, protonables (cation) en milieu légèrement acide, fixés sur le polymère qui jouent le rôle d'auxiliaires de passage transmembranaire.

L'intérêt de l'invention est que ces nouveaux complexes d'acide nucléique et de polymère substitué sont aussi ou plus efficaces sans auxiliaires de passage transmembranaire que les complexes d'acide nucléique et de polymère non substitué ou substitué par des agents réduisant le nombre de charges du polymère (donc son interaction avec l'acide nucléique) en présence d'auxiliaires de passage transmembranaire.

Dans le cas de la chloroquine et des peptides perméabilisants et/ou fusiogènes, ces derniers sont de petites molécules qui diffusent rapidement s'ils ne sont pas liés covalemment aux complexes d'acide nucléique et de polymère substitué.

L'intérêt de l'invention est que ces nouveaux complexes d'acide nucléique et de polymère substitué sont aussi efficaces (voire plus efficaces) en présence de sérum qu'en absence de sérum pour transfecter les cellules.

L'invention concerne un complexe entre au moins un acide nucléique (chargé négativement) et au moins un conjugué polymérique chargé positivement, la liaison entre l'acide nucléique et le conjugué polymérique étant de nature électrostatique, le conjugué polymérique contenant un polymère formé de motifs monomères portant des fonctions $NH_3^+$ libres, et étant tel que :

- les fonctions $NH_3^+$ libres des susdits motifs monomères sont substituées dans un rapport d'au moins 10%, par des résidus protonables dans un milieu dont le pH est inférieur à celui du plasma ou du sérum, lesdits résidus entraînant dans un milieu dont le pH est inférieur à celui du plasma ou du sérum une déstabilisation des membranes cellulaires, notamment la membrane des vésicules d'endocytose, et/ou des endosomes,
- les susdits résidus possédant en outre les propriétés suivantes :
  . ils comportent un groupe fonctionnel leur permettant d'être fixés au susdit polymère,
  . ils ne sont pas actifs en tant que signal de reconnaissance reconnu par un récepteur membranaire cellulaire,
  . ils appartiennent à la famille des composés comportant un noyau imidazole,

- . ils appartiennent à la famille des quinolines,
- . ils appartiennent à la famille des ptérines,
- . ils appartiennent à la famille des pyridines.

sous réserve que l'ensemble des fonctions $NH_3^+$ libres soit d'au moins 30% du nombre des motifs monomères du squelette polymérique du susdit conjugué polymérique,
et sous réserve que le conjugué polymérique soit différent du conjugué polylysine - (dithiopyridyl)propionate.
Selon un mode de réalisation, les complexes de l'invention sont tels que les résidus protonables dans un milieu dont le pH est inférieur à celui du plasma ou du sérum comportent au moins une fonction $NH_3^+$ libre.
Selon un autre mode de réalisation, les complexes de l'invention sont tels que des molécules constituant un signal de reconnaissance reconnu par un récepteur membranaire cellulaire sont présentes :

- . soit par substitution de certaines des fonctions $NH_3^+$ libres des motifs monomères,
- . soit sur certains des résidus entraînant une déstabilisation des membranes cellulaires,
- . soit par substitution de la fonction $NH_3^+$ libre des résidus entraînant une déstabilisation des membranes cellulaires.

Selon un autre mode de réalisation, les complexes de l'invention sont tels que les fonctions $NH_3^+$ libres des motifs monomères sont substituées dans un rapport d'environ 15% à environ 45%.
Par déstabilisation des membranes, on entend une modification de la membrane qui conduit soit à l'augmentation de sa perméabilité vis-à-vis de molécules de soluté de faible masse moléculaire et éventuellement de haute masse moléculaire (y compris des acides nucléiques, des plasmides ou des complexes), soit à la fusion avec une autre membrane.
La perméabilité membranaire peut être mesurée par microscopie de fluorescence de la façon suivante :
Les cellules adhérentes sont incubées à 37°C pendant 15 à 30 minutes avec 0.5 ml de milieu de culture DMEM avec sérum contenant 5 mg/ml de dextran (Mw 4000) marqué avec de l'isothiocyanate de fluorescéine (FTC-Dextran) et un complexe ADN/polylysine histidylée. Les cellules sont ensuite lavées et incubées à 37°C pendant 15 à 30 minutes avec du milieu de culture contenant 10% de sérum bovin foetal. Les cellules sont ensuite fixées pendant 5 minutes dans une solution de tampon phosphate salin contenant 4% de *p*-formaldéhyde et la fluorescence est analysée avec un microscope de fluorescence à plan confocal (MRC600 BioRad). En absence de perméabilisation membranaire, la fluorescence provenant du FTC-Dextran est localisée exclusivement dans des vésicules. En présence d'un agent de perméabilisation membranaire, la fluorescence provenant du FTC-Dextran est en outre observée de manière diffuse dans le cytosol et le noyau des cellules.
La fusion des membranes en présence de complexes ADN/polylysine histidylée se mesure facilement dans des systèmes modèles tels que les liposomes en utilisant une méthode de mélange lipidique comme celle décrite dans Struck D.K. *et al*. (Use of resonance energy transfer to monitor membrane fusion. (1981) Biochemistry 20:4093-4099). Brièvement, on utilise des liposomes constitués de dioléoyl-phosphatidylcholine (DPOC) où sont insérés dans leur membrane du N-(7-nitrobez-2-oxa-1,3-diazol-4-yl)phosphatidyl éthanolamine (NBD-PE) et de l'octadecylrhodamine (R18) comme marqueur fluorescent et des liposomes sans marqueurs fluorescents. On mesure la fluorescence du NBD (λex = 470 nm ; λem = 530 nm) en absence et en présence de complexes ADN/polylysine histidylée à différents pH. La fusion des liposomes induit une diminution de la fluorescence du NBD par suite d'une diminution du transfert d'énergie entre la rhodamine et le NBD.
Ces nouveaux complexes d'acide nucléique et de polymère substitué contenant des groupements faiblement basiques, protonables (cation) en milieu légèrement acide en présence de sérum, sont donc mieux adaptés pour un transfert de gènes *in vivo* que les complexes ADN/polylysine ou ADN/polylysine gluconoylée qui ne sont actifs qu'en présence d'auxiliaires tels que la chloroquine ou des peptides fusiogènes et/ou perméabilisants.
Les résidus entraînent la déstabilisation des membranes cellulaires grâce à leur propriété d'être protonables en milieu acide.
Les résidus entraînant la déstabilisation des membranes cellulaires sont des capteurs de protons qui limitent l'acidification des endosomes et, en conséquence, défavorisent la fusion entre les endosomes tardifs et les liposomes. On rappelle que les lysosomes sont des vésicules contenant un grand nombre d'hydrolases et que ces lysosomes sont très efficaces pour dégrader les macromolécules biologiques en général et les acides nucléiques en particulier.
L'expression "milieu faiblement acide" désigne un milieu dont le pH est inférieur à celui du plasma ou du sérum, par exemple un pH inférieur à 7,4.
L'expression selon laquelle les résidus "ne sont pas actifs en tant que signal de reconnaissance reconnu par un récepteur membranaire cellulaire" signifie que, d'une part, à ce jour, il n'y a pas de récepteurs connus qui soient spécifiques de ces résidus et, d'autre part, que ces résidus ne sont pas utilisés en tant que ligands.
Une molécule ou un complexe moléculaire est actif en tant que signal de reconnaissance, s'il peut être reconnu sélectivement par un récepteur, c'est-à-dire qu'il joue le rôle d'un ligand, d'un agoniste ou d'un antagoniste.

Par signal de reconnaissance reconnu par un récepteur membranaire cellulaire, on désigne généralementun ligand (molécule ou complexe moléculaire) capable d'être reconnu sélectivement par ledit récepteur (affinité ligand-récepteur ≥ $10^3$l/mole).

L'invention concerne notamment un complexe entre au moins un acide nucléique (chargé négativement) et au moins un conjugué polymérique chargé positivement, la liaison entre l'acide nucléique et le conjugué polymérique étant de nature électrostatique, le conjugué polymérique contenant un polymère formé de motifs monomères portant des fonctions $NH_3^+$ libres, et étant tel que :

- les fonctions $NH_3^+$ libres des susdits motifs monomères sont substituées dans un rapport d'au moins 10%, avantageusement d'environ 15% à environ 45%, notamment de 35%, ce rapport étant déterminépar exemple par résonance magnétique nucléaire, par des résidus protonables en milieu faiblement acide entraînant en milieu faiblement acide une déstabilisation des membranes cellulaires, notamment la membrane des vésicules d'endocytose et/ou des endosomes,
- les susdits résidus possédant en outre les'propriétés suivantes :
  - . ce sont des bases dont le pK en milieu aqueux est inférieur à 8, de sorte qu'une proportion supérieure à 50% de ces bases liée à un polymère cationique ne soit pas protonée en milieu neutre de pH7,4,
  - . ils comportent un groupe fonctionnel leur permettant d'être fixés au susdit polymère,
  - . ils ne sont pas actifs en tant que signal de reconnaissance reconnu par un récepteur membranaire cellulaire,
  - . ils peuvent comporter au moins une fonction $NH_3$+ libre,
  - . ils appartiennent à la famille des composés comportant un noyau imidazole
  - . ils appartiennent à la famille des quinolines,
  - . ils appartiennent à la famille des utérines,
  - . ils appartiennent à la famille des pyridines
- les fonctions $NH_3^+$ libres des susdits motifs monomères pouvant être également substituées par des résidus non chargés entraînant une diminution des charges positives par rapport au même conjugué polymérique non substitué, facilitant le relargage de l'acide nucléique par au cours de la dissociation du complexe,
- les susdits résidus non chargés possédant en outre les propriétés suivantes:
  - . ils comportent au moins un groupe hydroxyle,
  - . ils ne sont pas actifs en tant que signal de reconnaissance reconnu par un récepteur membranaire cellulaire,
- des molécules constituant un signal de reconnaissance reconnu par un récepteur membranaire cellulaire étant éventuellement présents :
  - . soit par substitution de certaines fonctions $NH_3$+ libres des susdits motifs monomères (par exemple ε-$NH_3$+ de la lysine),
  - . soit sur certains des susdits résidus non chargés entraînant une diminution de charge (par exemple gluconoyle), et notamment sur les groupes hydroxyles des susdits résidus non chargés entraînant une diminution de charge,
  - . soit sur certains des susdits résidus entraînant une déstabilisation des membranes cellulaires (par exemple imidazole acétyl), soit par substitution de la fonction $NH_3$+ éventuelle, libre des susdits résidus entraînant une déstabilisation des membranes cellulaires (par exemple histidine),

sous réserve que l'ensemble des fonctions $NH_3^+$ libres soit d'au moins 30% du nombre des motifs monomères du squelette polymérique du susdit conjugué polymérique.

L'invention concerne également un complexe entre au moins un acide nucléique (chargé négativement) et au moins un conjugué polymérique chargé positivement, la liaison entre l'acide nucléique et le conjugué polymérique étant de nature électrostatique, le conjugué polymérique contenant un polymère formé de motifs monomères portant des fonctions $NH_3^+$ libres, et étant tel que :

- les fonctions $NH_3^+$ libres des susdits motifs monomères sont substituées dans un rapport d'au moins 10%, avantageusement d'environ 15% à environ 45 %, notamment de 35 %, ce rapport étant déterminé par exemple par résonance magnétique nucléaire, par des résidus protonables en milieu faiblement acide entraînant en milieu faiblement acide une déstabilisation des membranes cellulaires, notamment la membrane des vésicules d'endocytose,
- les susdits résidus possédant en outre les propriétés suivantes :

. ils appartiennent à la famille des composés comportant un noyau imidazole,
. ils appartiennent à la famille des quinolines,
. ils appartiennent à la famille des ptérines,
. ils appartiennent à la famille des pyridines,
. les susdits résidus comportent un groupe fonctionnel leur permettant d'être fixés au susdit polymère,
. ils peuvent comporter au moins une fonction $NH_3^+$ libre,
. ils ne sont pas actifs en tant que signal de reconnaissance reconnu par un récepteur membranaire cellulaire,

- les fonctions $NH_3^+$ libres des susdits motifs monomères pouvant être également substituées par au moins une molécule qui constitue un signal de reconnaissance reconnu par un récepteur membranaire cellulaire, et/ou par des résidus non chargés entraînant une diminution des charges positives par rapport au même conjugué polymérique non substitué, facilitant le relargage de l'acide nucléique au cours de la dissociation du complexe, sous réserve que l'ensemble des susdits résidus contienne au moins 30% de fonctions $NH_3^+$ libres,
- les fonctions $NH_3^+$ libres des susdits motifs monomères pouvant être également substituées par au moins une molécule constituant un signal de reconnaissance reconnu par un récepteur membranaire cellulaire, et/ou par des résidus non chargés entraînant une diminution des charges positives par rapport au même conjugué polymérique non substitué, facilitant le relargage de l'acide nucléique par la dissociation du complexe,
- les susdits résidus non chargés possédant en outre les propriétés suivantes :

. ils comportent au moins un groupe hydroxyle,
. ils ne sont pas actifs en tant que signal de reconnaissance reconnu par un récepteur membranaire cellulaire,

- des molécules constituant un signal de reconnaissance reconnu par un récepteur membranaire cellulaire étant éventuellement présentes :

. soit par substitution de certaines des fonctions $NH_3^+$ libres des susdits motifs monomères (par exemple $\varepsilon$-$NH_3^+$ de la lysine),
. soit sur certains des susdits résidus non chargés entraînant une diminution de charge (par exemple gluconoyle) et notamment sur les groupes hydroxyles des susdits résidus non chargés entraînant une diminution de charge,
. soit sur certains des susdits résidus entraînant une déstabilisation des membranes cellulaires (par exemple imidazole acétyl),
. soit par substitution de la fonction $NH_3^+$ éventuelle, libre des susdits résidus entraînant une déstabilisation des membranes cellulaires (par exemple histidine),

sous réserve que l'ensemble des fonctions $NH_3^+$ libres soit d'au moins 30% du nombre des motifs monomères du squelette polymérique du susdit conjugué polymérique.

Les signaux de reconnaissance peuvent également entraîner une diminution des charges positives du conjugué polymérique lorsqu'ils sont eux-mêmes neutres ou acides et qu'ils sont liés au polymère par substitution d'une fonction $NH_3^+$ entraînant la perte de la charge +.

Les signaux de reconnaissance sont des molécules de petite masse moléculaire (< 5000 daltons).

Le nombre de molécules de signal de reconnaissance fixé sur le polymère modifié peut être :

- pour une molécule signal de très haute affinité vis-à-vis de son récepteur (Ka > $10^7$ l/mole), d'environ 0,5 à 5, avantageusement 1 molécule pour environ 10 000 motifs monomères de polymère substitué soit 1 molécule pour environ 50 molécules de polymère substitué;
- pour une molécule signal de haute affinité vis-à-vis de son récepteur (Ka entre $10^5$ l/mole et $10^7$ l/mole), d'environ 0,5 à environ 10, avantageusement 1 molécule pour environ 200 motifs monomères de polymère substitué soit 1 molécule pour environ 1 molécule de polymère substitué;
- pour une molécule signal de moyenne affinité vis-à-vis de son récepteur (Ka < $10^5$ l/mole), d'environ 10 à environ 100, avantageusement 50 molécules pour environ 200 motifs monomères de polymère substitué soit 50 molécules pour environ 1 molécule de polymère substitué.

La famille des quinolines est représentée par la formule suivante :

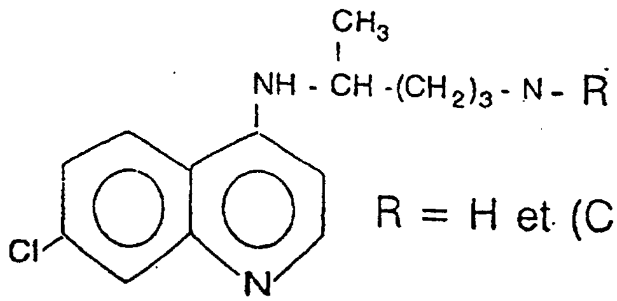

R = H et $(CH_2)_n$-$CO_2H$

dans laquelle n vaut de 1 à 10, de préférence de 1 à 3.

La famille des ptérines est représentée par la formule suivante :

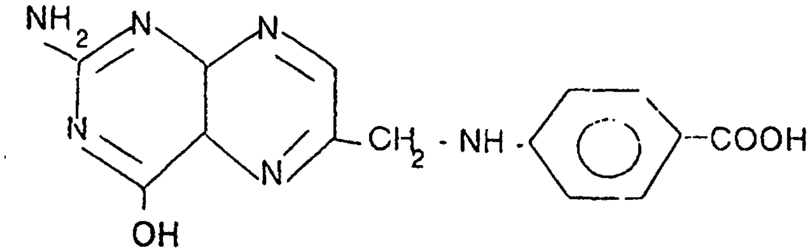

La famille des pyridines est représentée par les formules suivantes :

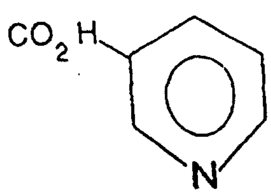

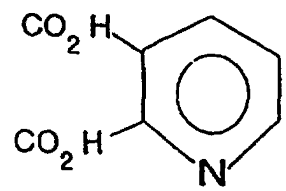

L'invention concerne également un complexe dans lequel les résidus entraînant en milieu faiblement acide une déstabilisation des membranes cellulaires sont

- des alkylimidazoles dans lequel le radical alkyle comporte de 1 à 10, notamment de 2 à 6 atomes de carbone, et dans lequel un seul des atomes d'azote du noyau imidazole est substitué,
- ou des quinolines de formule :

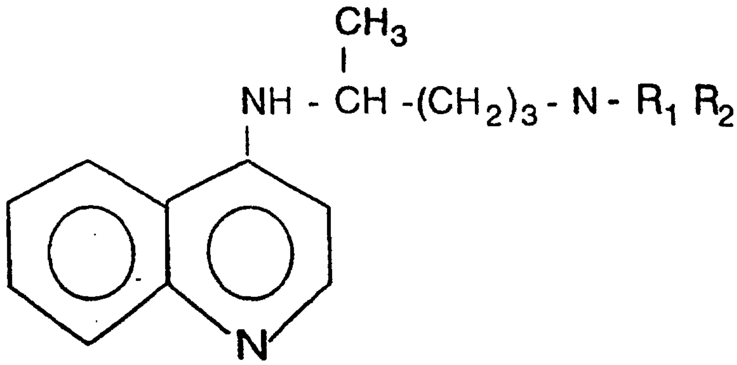

dans laquelle $R_1$ représente H et R2 représente $(CH_2)n-CO_2-H$, n étant un nombre entier variant de 1 à 10, et de préférence valant de 1 à 3.

L'invention concerne également un complexe dans lequel les résidus entraînant une déstabilisation des membranes cellulaires sont choisis parmi : histidine, 4-carboxyméthyl-imidazole, 3-(1-méthyl-imidazol-4yl)-alanine, 3-(3-méthyl-imidazol-4yl)-alanine, 2-carboxy-imidazole, histamine, acide 3-(imidazol-4yl)-L-lactique, 2-(1-méthyl-imidazol-4yl) éthylamine, 2-(3-méthyl-imidazol-4yl)éthylamine, β-alanyl-histidine-(carnosine), 7-chloro-4(amino-1-methylbutylamino)-quinoline, $N^4$-(7-chloro-4-quinolinyl)-1,4-pentanediamine, 8-(4-amino-1-méthylbutylamino)-6-méthoxy quinoline (primaquine), $N^4$-(6-méthoxy-8-quinolinyl)1,4-pentanediamine, acide quininique, acide quinoline carboxylique, acide ptéroïque, acide nicotinique, acide quinolinique,
et dans lequel

- la fonction $NH_3^+$ éventuelle, libre des susdits résidus (par exemple histidine) peut être également substituée par une molécule qui constitue un signal de reconnaissance reconnu par un récepteur membranaire cellulaire,

sous réserve que l'ensemble des fonctions $NH_3^+$ libres soit d'au moins 30% du nombre des motifs monomères du squelette polymérique du susdit conjugué polymérique.

L'invention concerne un complexe entre au moins un acide nucléique (chargé négativement) et au moins un conjugué polymérique chargé positivement, la liaison entre l'acide nucléique et le conjugué polymérique étant de nature électrostatique, le conjugué polymérique contenant un polymère formé de motifs monomères portant des fonctions $NH_3^+$ libres, notamment des résidus de lysine ou d'ornithine, et étant tel que :

- les fonctions $NH_3^+$ libres des susdits motifs monomères sont substituées dans un rapport d'au moins 10%, avantageusement d'environ 15% à environ 45%, notamment de 35%, par des résidus entraînant en milieu faiblement acide une déstabilisation des membranes cellulaires,
- les susdits résidus possédant en outre les propriétés suivantes :
  - . ils comportent un noyau imidazole,
  - . ils peuvent comporter au moins une fonction $NH_3^+$ libre,
  - . ils ne sont pas actifs en tant que signal de reconnaissance,
- les fonctions $NH_3^+$ libres restantes des susdits motifs monomères étant également substituées à raison d'environ 1% à environ 60% par une molécule qui constitue un signal de reconnaissance reconnu par un récepteur membranaire cellulaire, ce signal de reconnaissance ayant une masse moléculaire inférieure à 5000, ce signal de reconnaissance pouvant être présent à raison d'une molécule pour environ 200 motifs du conjugué polymérique ou d'environ 60 molécules pour environ 200 motifs du conjugué polymérique,

sous réserve que l'ensemble des fonctions $NH_3^+$ libres soit d'au moins 30% du nombre des motifs monomères du squelette polymérique du susdit conjugué polymérique.

L'expression "non actifs en tant que signal de reconnaissance" signifie que, d'une part, à ce jour, il n'y a pas de récepteurs connus qui soient spécifiques de ces résidus et, d'autre part, que ces résidus ne sont pas utilisés en tant que ligands.

L'invention concerne également un complexe dans lequel le polymère contient un groupement polymérique de formule (I) suivante :

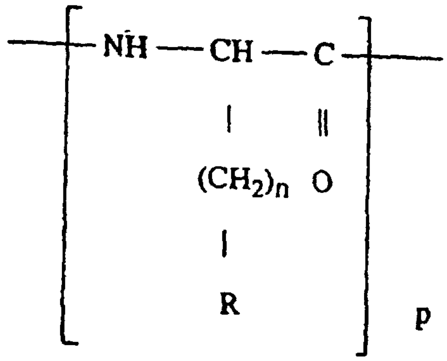

(I)

dans laquelle :

- p est un nombre entier variant de 15 à 900, de préférence de 100 à 300,
- n est un nombre entier variant de 1 à 6, et vaut de préférence 4,
- ce groupement polymérique contient des radicaux R parmi lesquels :

  . 10% à 45% du nombre de radicaux R représentant un résidu comportant un noyau imidazole et éventuellement une fonction $NH_3^+$ libre, notamment un résidu histidyle, R pouvant être représenté par la formule :

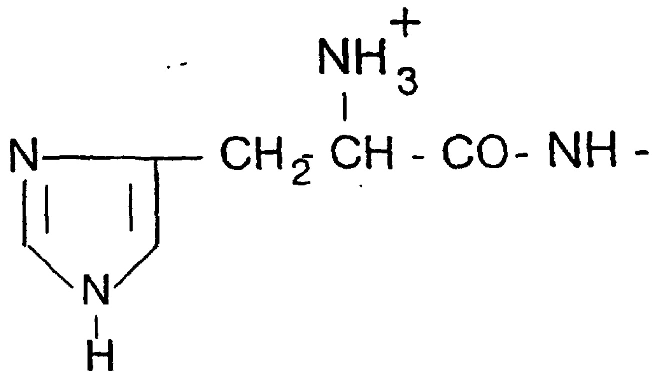

la fonction $NH_3^+$ éventuelle des susdits résidus pouvant être également substituée par une molécule qui constitue un signal de reconnaissance,

. 10% à 90% du nombre de radicaux R, représentant les $\bar{\omega}$-amino $NH_3^+$ libres, et étant éventuellement substitué à raison de 0 à 50% par une molécule qui constitue un signal de reconnaissance, notamment à raison de 0 à 60, avantageusement de 1 molécule pour environ 200 motifs, ou à raison de 2 à 100, avantageusement de 50 molécules pour environ 200 motifs, et/ou

. R pouvant en outre être constitué de 0 à 45% par un groupe NH-CO-$(CHOH)_m$-$R_1$, notamment un reste dihydroxypropionoylamido, érythronoylamido, thréonoylamido, ribonoylamido, arabinoylamido, xylonoylamido, lyxonoylamido, gluconoylamido, galactonoylamido, mannonoylamido, glycoheptonoylamido, glycooctonoylamido, m est un nombre entier de 2 à 15, de préférence de 2 à 7, $R_1$ représente H ou un radical alcoyle de 1 à 15 atomes de carbone, notamment $CH_3$, ces radicaux pouvant être substitués par une molécule qui constitue un signal de reconnaissance, sous réserve que l'ensemble des fonctions $NH_3^+$ libres soit d'au moins 30% du nombre des motifs monomères du squelette polymérique du susdit conjugué polymérique.

Dans cette classe de complexes de l'invention, le polymère est de la polylysine ou de la polyornithine.

Comme le montrent les exemples, les cellules HepG2 (hépatocarcinome humain) sont efficacement transfectées par de la polylysine substituée par 70 résidus histidyle.

La polylysine substituée par 30 ± 10% d'histidine a permis de transfecter différentes cellules (humaines et murines) avec une grande efficacité, modulée selon le type cellulaire et le promoteur utilisé.

L'invention a également pour objet un complexe dans lequel le polymère comprend un groupement polymérique de formule (II) suivante :

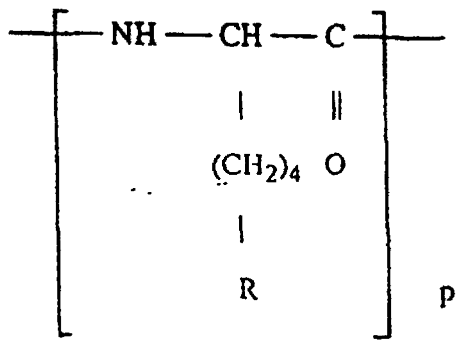

dans laquelle :

- p a les significations indiquées ci-dessus,
- 10% à 45% du nombre de radicaux R représentent un résidu comportant un noyau imidazole et éventuellement une fonction $NH_3^+$ libre, notamment un résidu histidyle, R pouvant être représenté par la formule

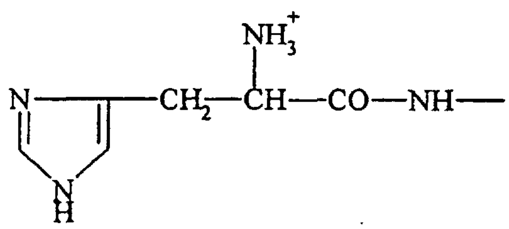

les fonctions $NH_3^+$ des susdits résidus pouvant être également substituées par une molécule qui constitue un signal de reconnaissance,
- le reste des radicaux, c' est-à-dire 30% à 90% du nombre de radicaux R, représentant les $\bar{\omega}$-amino $NH_3^+$ , et de 0% à 45% des radicaux R pouvant être substitués par une molécule qui constitue un signal de reconnaissance reconnu par un récepteur membranaire cellulaire,

sous réserve que l'ensemble des fonctions $NH_3^+$ libres soit d' au moins 30% du nombre des motifs monomères du squelette polymérique du susdit conjugué polymérique.

L' invention a également pour objet un complexe qui se caractérise en ce que le signal de reconnaissance est choisi parmi:

A) - des osides simples ou complexes reconnus par des lectines membranaires, et choisis parmi: ,

a. Asialo-oligoside de type triantenné lactosamine: récepteur d'asialoglycoprotéine

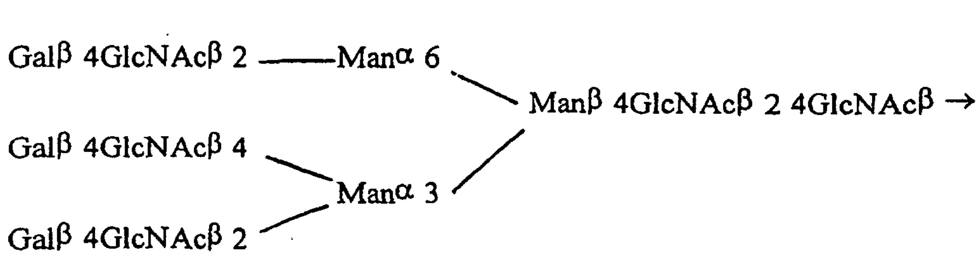

b. Asialo oligoside de type lactosamine tetraantenné : récepteur d'asialoglycoprotéine

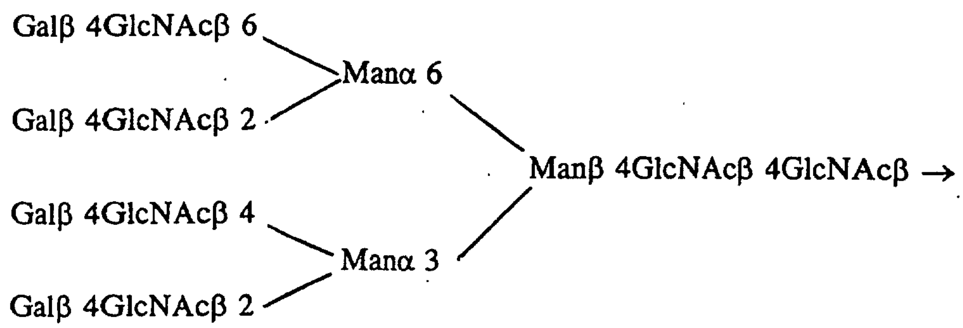

c. Lewis x:

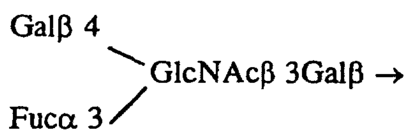

d. Lewis x sialyl: LECAM 3/2

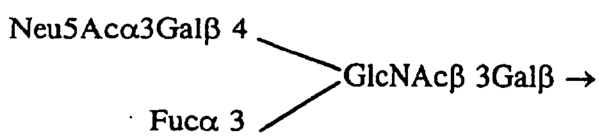

e. Dérivé de Lewis x sulfaté (HNK1):

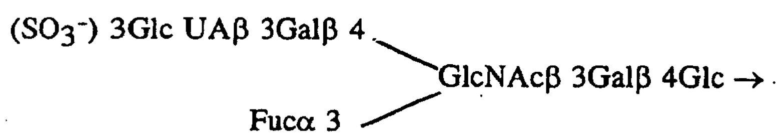

f. Oligomannoside: récepteur du mannose

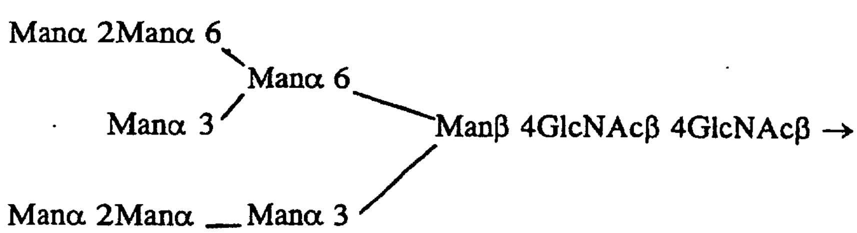

g. Oligomannoside phosphorylé: récepteur de mannose 6 phosphate

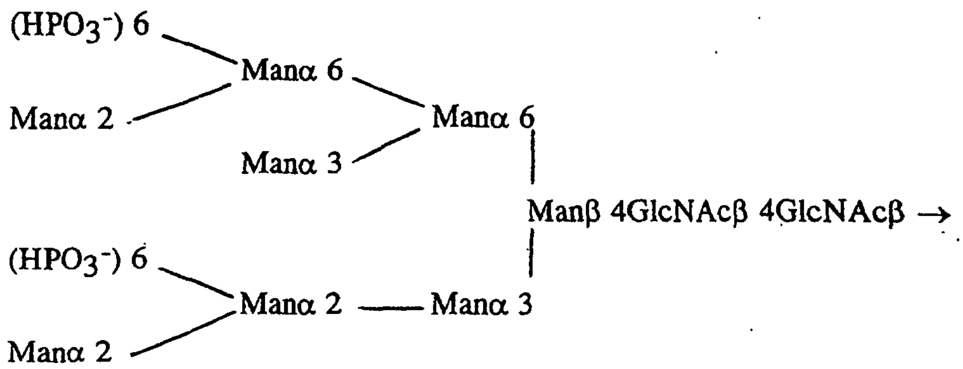

h. Oligosaccharide de type lactosamine sulfatée: récepteur de GalNAc 4 sulfaté

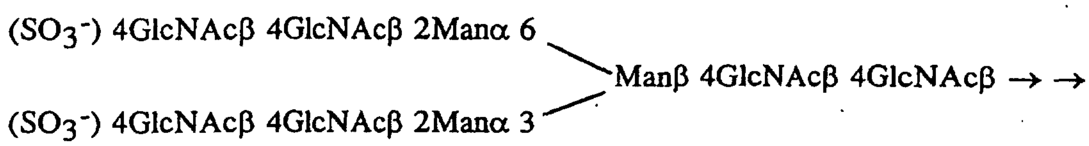

B) des peptides

a. peptides anti-inflammatoires ou certains de leurs fragments reconnus par des récepteurs de la paroi vasculaire, tels que

- polypeptide vasodilatateur intestinal (VIP)
  HSDAVFTDNYTRLRKQMAVKKYLNSILN-$NH_2$
- polypeptide atrial natriurétique (ANP)
  SLRRSSCFGGRMDRIGAQSGLGCNSFRY
- lipocortine
  HDMNKVLDL
- bradykinine
  RPPGFSPFR;

b. peptides ligands des intégrines, tels que les peptides contenant la séquence RGD, ligand de la fibronectine;

c. facteurs chimiotactiques, tels que les formyl peptides et leurs antagonistes:

FMLP, (N-formyl-Met-Leu-Phé);

d. hormones peptidiques tels que
l'α-MSH: Ac-SYSMEHFRWGKPV-$NH_2$ et leurs antagonistes.

C) Métabolites naturels tels que:

- la biotine,
- la carnitine.
- le tétrahydrofolate et l'acide folique pouvant être à la fois un signal de reconnaissance vis-à-vis de certaines cellules possédant les récepteurs appropriés et un déstabilisateur des membranes cellulaires.

L'invention concerne également un complexe qui se caractérise en ce que l'acide nucléique peut être choisi parmi:

a) des gènes marqueurs, tels que

- gènes contenant la luciférase,
- protéine verte de la méduse *Aequorea victoria*,
- gènes contenant la β-galactosidase,
- gènes contenant la chloramphénicol acétyl transférase,
- gènes conférant la résistance à un antibiotique, tels que l'hygromycine, la néomycine etc...;

b) des gènes à visée thérapeutique, tels que

- récepteurs des lipoprotéines de faible densité, déficient dans les cas d' hypercholestérolémie,
- facteurs de coagulation: facteurs VIII et IX,
- phénylalanine-hydroxylase (phénylcétonurie),
- adénosine désaminase (immunodéficience ADA),
- enzymes lysosomiques, telles que la β-glucosidase dans le cas de la maladie de Gaucher,
- dystrophine et minidistrophine (myopathie),
- tyrosine hydroxylase (Parkinson),
- facteurs de croissance des neurones (Alzheimer),
- CFTR cystic-fibrosis transmembrane conductance regulator (mucoviscidose),
- alphal-antitrypsine,
- cytokines (interleukines, TNF facteur de nécrose des tumeurs),
- thymidine kinase du virus Herpes simplex,
- protéines du MHC, système majeur d'histocompatibilité, en particulier les HLA-B7,
- cytosine désaminase,
- gènes codant pour des ARN sens et antisens,
- gènes codant pour des ribozymes,

c) des gènes à visée vaccinale

- gènes codant pour des antigènes viraux (vaccination), par exemple :

  gène codant pour la nucléoprotéine du virus de la grippe.

L'invention a également pour objet un complexe dans lequel :

- le polymère, notamment la polylysine présente un degré de polymérisation d'environ 15 à environ 900, de préférence 200,
- les fonctions $NH_3^+$ libres des motifs lysine étant substituées dans un rapport de 35% par des résidus histidyle et éventuellement par une molécule constituant un signal de reconnaissance pour 1 à 50 résidus de lysine lorsque ladite molécule signal possède une affinité d'au moins $10^5$ l $mole^{-1}$ vis-à-vis du récepteur de la cellule que le complexe doit cibler ou éventuellement par 20 à 100 molécules de signal de reconnaissance pour 200 résidus de lysine lorsque ladite molécule signal possède une affinité inférieure à $10^5$ l $mole^{-1}$ vis-à-vis du susdit récepteur,
- l'acide nucléique présente une masse moléculaire d'environ $10^6$ à environ $10^8$, notamment de 3.106 à 30.106,
- le rapport entre le nombre moyen de paires de base de l'acide nucléique par molécule de motif de monomère, notamment la lysine est d'environ 0,2 à environ 6, de préférence d'environ 0,4 à environ 0,6.

S'agissant des affinités :

- pour une molécule signal de très haute affinité vis-à-vis de son récepteur (Ka > $10^7$ l/mole), d'environ 0,5 à 5, avantageusement 1 molécule pour environ 10 000 motifs monomères de polymère substitué soit 1 molécule pour environ 50 molécules de polymère substitué ;
- pour une molécule signal de haute affinité vis-à-vis de son récepteur (Ka entre $10^5$ l/mole et $10^7$ l/mole), d'environ 0,5 à environ 10, avantageusement 1 molécule pour environ 200 motifs monomères de polymère substitué soit 1 molécule pour environ 1 molécule de polymère substitué ;
- pour une molécule signal de moyenne affinité vis-à-vis de son récepteur (Ka < $10^5$ l/mole), d'environ 10 à environ 100, avantageusement 50 molécules pour environ 200 motifs monomères de polymère substitué soit 50 molécules pour environ 1 molécule de polymère substitué.

L'invention a également pour objet un conjugué polymérique chargé positivement, contenant des motifs portant des fonctions $NH_3^+$ libres, et étant tel que :

- les fonctions $NH_3^+$ libres des susdits motifs monomères sont substituées dans un rapport d'au moins 10%, avantageusement d'environ 15% à environ 45%, notamment de 35%, ce rapport étant déterminé par exemple par résonance magnétique nucléaire, par des résidus protonables en milieu faiblement acide entraînant en milieu faiblement acide une déstabilisation des membranes cellulaires, notamment la membrane des vésicules d'endocytose,
- les susdits résidus possédant en outre les propriétés suivantes :

  . ils comportent un groupe fonctionnel leur permettant d'être fixés au susdit polymère,
  . ils ne sont pas actifs en tant que signal de reconnaissance reconnu par un récepteur membranaire cellulaire,
  . ils peuvent comporter au moins une fonction $NH_3$+ libre,
  . ils appartiennent à la famille des composés comportant un noyau imidazole
  . ils appartiennent à la famille des quinolines,
  . ils appartiennent à la famille des utérines,
  . ils appartiennent à la famille des pyridines

- les fonctions $NH_3^+$ libres des susdits motifs monomères pouvant être également substituées par des résidus non chargés entraînant une diminution des charges positives par rapport au même conjugué polymérique non substitué, facilitant le relargage de l'acide nucléique par la dissociation du complexe,
- les susdits résidus non chargés possédant en outre les propriétés suivantes:

  . ils comportent au moins un groupe hydroxyle,
  . ils ne sont pas actifs en tant que signal de reconnaissance reconnu par un récepteur membranaire cellulaire,
  . les groupes hydroxyles des susdits résidus non chargés pouvant être substitués par au moins une molécule qui constitue un signal de reconnaissance reconnu par un récepteur membranaire cellulaire,

- des molécules constituant un signal de reconnaissance reconnu par un récepteur membranaire cellulaire étant éventuellement présents :

  . soit par substitution de certaines fonctions $NH_3^+$ libres des susdits motifs monomères (par exemple ε-$NH_3^+$ des lysines),
  . soit sur certains des susdits résidus non chargés entraînant une diminution de charge (par exemple gluconoyle), et notamment sur les groupes hydroxyles des susdits résidus non chargés, entraînant une diminution de charge,
  . soit sur certains des susdits résidus entraînant une déstabilisation des membranes cellulaires (par exemple imidazole acétyl),
  . soit par substitution de la fonction $NH_3^+$ éventuelle, libre des susdits résidus entraînant une déstabilisation des membranes cellulaires (par exemple histidine),

sous réserve que l'ensemble des fonctions $NH_3^+$ libres soit d'au moins 30% du nombre des motifs monomères du squelette polymérique du susdit conjugué polymérique.

L'invention concerne également un conjugué polymérique tel que défini ci-dessus ou contenant un groupement polymérique tel que défini ci-dessus.

Selon un mode de réalisation avantageux de l'invention, le conjugué polymérique est choisi parmi la polylysine histidylée substituée par du lactose, la polylysine histidylée substituée par un oligoside complexe tel que Lewis$^b$, la polylysine histidylée substituée par le peptide ANP ou la polylysine histidylée substituée par de la biotine.

La préparation des conjugués polymériques de l'invention peut se faire selon l'une des façons décrites dans les tableaux suivants :

Tableau I :

| Méthodes de préparation des conjugués polymériques avec des signaux de reconnaissance fixés sur certains motifs monomériques du polymère. | | | |
|---|---|---|---|
| On a indiqué par 1, 2, 3 l'ordre d'introduction respectif des résidus responsables de la déstabilisation, de ceux responsables de la diminution de charge et des signaux de reconnaissance sur le polymère. | | | |
| POLYMÈRE | | | |
| méthode | résidus | | |
| | responsables de la déstabilisation | responsables de la diminution de charge | signal de reconnaissance |
| I | 1 | - | 2 |
| II | 2 | - | 1 |
| III | 1 | 2 | 3 |
| IV | 1 | 3 | 2 |
| V | 2 | 1 | 3 |
| VI | 3 | 1 | 2 |
| VII | 2 | 3 | 1 |
| VIII | 3 | 2 | 1 |

Tableau II :

| Méthodes de préparation des conjugués polymériques avec des signaux de reconnaissance fixés sur certains résidus responsables de la déstabilisation des membranes. | | | |
|---|---|---|---|
| On a indiqué par 1, 2, 3 l'ordre d'introduction respectif des résidus responsables de la déstabilisation, de ceux responsables de la diminution de charge et des signaux de reconnaissance sur le polymère. | | | |
| POLYMERE | | | |
| méthode | résidus | | |
| | responsables de la déstabilisation | responsables de la diminution de charge | signal de reconnaissance |
| IX | 1 | - | 2 |
| X | 1 | 2 | 3 |
| XI | 1 | 3 | 2 |
| XII | 2 | 1 | 3 |

Tableau III :

| Méthodes de préparation des conjugués polymériques avec des signaux de reconnaissance fixés sur certains résidus responsables de la diminution de charge. | | | |
|---|---|---|---|
| On a indiqué par 1, 2, 3 l'ordre d'introduction respectif des résidus responsables de la déstabilisation, de ceux responsables de la diminution de charge et des signaux de reconnaissance sur le polymère. | | | |
| POLYMERE | | | |
| méthode | résidus | | |
| | responsables de la déstabilisation | responsables de la diminution de charge | signal de reconnaissance |
| XIII | 2 | 1 | 3 |
| XIV | 3 | 1 | 2 |
| XV | 1 | 2 | 3 |

Les résidus responsables de la déstabilisation des membranes sont choisis parmi: histidine, 4-carboxyméthyl-imidazole, 3-(1-méthyl-imidaxol-4yl)-alanine, 3-(1-méthyl-imidazol-4yl)-alanine, 2-carboxy-imidazole, histamine, - acide 3-(imidazol-4yl)-L-lactique, 2-(1-méthyl-imidazol-4yl)-éthylamine, 2-(3-méthyl-imidazol-4yl)-éthylamine, β-alanylhistidine, 7-chloro-4(amino-1-méthylbutylamino)-quinoline, $N^4$-(7-chloro-4-quinolinyl)-1,4-pentanediamine, 8-(4-amino-1-méthylbutylamino)-6-méthoxy-quinoline, $N^4$-(6-méthoxy-8-quinolinyl)-1,4-pentanediamine, acide quininique, acide quinoline carboxylique, acide ptéroïque, acide nicotinique, acide quinolinique.

Les résidus responsables de la diminution de charge sont choisis parmi: dihydroypropionyle, érythronoyle, thréonoyle, ribonoyle, arabinoyle, xylonoyle, lyxonoyle, gluconoyle, galactonoyles, mannonoyle, glycoheptonoyle, glycooctonoyle.

Les signaux de reconnaissance sont choisis parmi : des osides, des oligosides, des peptides, des métabolites, des agonistes, des antagonistes.

A titre d'exemple nous décrivons différentes méthodes des tableaux :

*I) Les signaux de reconnaissance sont fixés sur certains motifs monomériques du polymère après l'introduction des résidus entraînant une déstabilisation des membranes cellulaires de la façon suivante :*

A) Méthode I

polylysine nicotinylée

a) les motifs monomères du polymère comportant une fonction NH3+ libre sont partiellement substitués par des résidus entraînant une déstabilisation des membranes cellulaires. Par exemple, la polylysine (notamment sous forme de *p*-toluène sulfonate) est dissoute dans un solvant organique (notamment le diméthylsulfoxyde) en présence d'une base (notamment la diisopropyléthylamine), de molécules entraînant une déstabilisation des membranes cellulaires (notamment l'acide nicotinique) et d'un agent de couplage (notamment l'hexafluorophosphate de benzotriazolyl-N-oxytrisdimethylaminophosphonium).

b) Les signaux de reconnaissance sont liés à certains groupements ε-aminés des résidus lysyles du polymère.

A titre d'exemple de fixation des signaux de reconnaissance sur la polylysine nicotinylée, on indique ci-après la fixation d'osides ou d'oligosides.

1) fixation d'osides.

Des osides simples sous la forme de dérivés phénylisothiocyanates sont fixés sur certaines fonctions ε-aminées des résidus lysyles libres de la polylysine comme précédemment décrit dans *Midoux et al*., (Nucleic Acids Res. 1993, 21: 871-878).

2) fixation d'oligosides.

Les oligosides complexes tels que les asialo-oligosides bi-, tri- ou tétraantennés ou Lewis sont obtenus sous forme de dérivés phénylisothiocyanates de glycopeptides selon une méthode décrite dans Monsigny *et al*., (Brevet Français 9407738 (Monsigny, M., Sdiqui, N., Roche, A.C. and Mayer, R. 1994 Nouveaux dérives d'oligosides, leur procédé de préparation et leurs applications) et Sdiqui *et al*., (1995 New synthesis of glyco-amino acid conjugates. Carbohyd. Letters 1, 269-275). Les glycopeptides sous la forme de dérivés phénylisothiocyanates sont fixés sur certaines fonctions ε-aminés des résidus lysyles libres de la polylysine comme précédemment décrit dans Midoux *et al*., (Nucleic Acids Res. 1993, 21 : 871-878).

polylysine histidylée

a) les motifs monomères du polymère comportant une fonction NH3+ libre sont partiellement substitués par des résidus comportant une fonction permettant la fixation ultérieure d'autres molécules telles que celles qui constitueront un signal de reconnaissance, par exemple, après réaction en milieu organique avec un ester N-hydroxysuccinimide de l'acide dithiopyridine propionate ou de ses dérivés.

b) les motifs monomères du polymère comportant une fonction NH3+ libre sont ensuite partiellement substitués par des résidus entraînant une déstabilisation des membranes cellulaires, par exemple, après réaction en milieu organique avec l'histidine dont le groupe αNH3+ et le groupe NH du noyau imidazole sont protégées par du tertiobutyloxycarbonyle en présence d'un agent de couplage tel que l'hexafluorophosphate de benzotriazolyl-N-oxytrisdimethylaminophosphonium. Après réaction et purification, les fonctions aminées des résidus histidyles du polymère obtenu sont déprotégées.

c) Les signaux de reconnaissance sont liés aux groupements dithiopyridyles du polymère.

A titre d'exemple de fixation des signaux de reconnaissance sur la polylysine histidylée, on indique ci-après la fixation d'osides ou d'oligosides.

1) fixation d'osides.

Les osides simples dérivés en glycopeptides avec une fonction dithiopyridyle (*pyro*glutamyl-NH-$(CH2)_2$-S-S-pyridine) selon une méthode décrite dans Monsigny *et al*., (Brevet Français 9407738 (Monsigny, M., Sdiqui, N., Roche, A.C. and Mayer, R. 1994 Nouveaux dérives d'oligosides, leur procédé de préparation et leurs applications) et Sdiqui *et al*., (1995 New synthesis of glyco-amino acid conjugates. Carbohyd. Letters 1:269-275) sont réduits et fixés en milieu aqueux tamponné à pH neutre sur les fonctions dithiopyridyles du polymère.

2) fixation d'oligosides

Les oligosides complexes tels que les asialo-oligosides bi-, tri- ou tétraantennés ou Lewis dérivés en glycopeptides avec une fonction dithiopyridyle (*pyro*glutamyl-NH-$(CH2)_2$-S-S-pyridine) selon une méthode décrite dans Monsigny *et al*., (Brevet Français 9407738 (Monsigny, M., Sdiqui, N., Roche, A.C. and Mayer, R. 1994 Nouveaux dérives d'oligosides, leur procédé de préparation et leurs applications) et Sdiqui *et al*., (1995 New synthesis of glyco-amino acid conjugales. Carbohyd. Letters 1:269-275) sont réduits et fixés en milieu aqueux tamponné à pH neutre sur les fonctions dithiopyridyles du polymère.

B) Méthode III

polylysine nicotinylée et gluconoylée

a) les motifs monomères du polymère comportant une fonction NH3+ libre sont partiellement substitués par des résidus entraînant une déstabilisation des membranes cellulaires. Par exemple la polylysine gluconoylée est dissoute dans un solvant organique (notamment le diméthylsulfoxyde) en présence d'une base (notamment la diisopropyléthylamine), de molécules entraînant une déstabilisation des membranes cellulaires (notamment l'acide nicotinique) et d'un agent de couplage (notamment l'hexafluorophosphate de benzotriazolyl-N-oxytrisdimethylaminophosphonium).
b) les motifs monomères du polymère comportant une fonction NH3+ encore libre sont ensuite partiellement substitués par des résidus non chargés entraînant une diminution de charge. S'agissant de la fixation des résidus entraînant la diminution de charge, par exemple un sel de polylysine (notamment sous forme de *p*-toluène sulfonate) est dissous dans un solvant organique (notamment le diméthylsulfoxyde) en présence d'une base (notamment la diisopropyléthylamine) et d'un acide organique hydroxylé activé (notamment la δ-gluconolactone).
c) Les signaux de reconnaissance sont liés à certains groupements ε-aminés des résidus lysyles du polymère.

A titre d'exemple de fixation des signaux de reconnaissance sur la polylysine histidylée, on indique ci-après la fixation d'osides ou d'oligosides.

1) fixation d'osides.

Des osides simples sous la forme de dérivés phénylisothiocyanates sont fixés sur certaines fonctions ε-aminées des résidus lysyles libres de la polylysine comme précédemment décrit dans Midoux *et al*., (Nucleic Acids Res. 1993, 21: 871-878).

2) fixation d'oligosides.

Les oligosides complexes tels que les asialo-oligosides bi-, tri- ou tétraantennés ou Lewis sont obtenus sous forme de dérivés phénylisothiocyanates de glycopeptides selon une méthode décrite dans Monsigny *et al*., (Brevet Français 9407738 (Monsigny, M., Sdiqui, N., Roche, A.C. and Mayer, R. 1994 Nouveaux dérives d'oligosides, leur procédé de préparation et leurs applications) et Sdiqui *et al*., (1995 New synthesis of glyco-amino acid conjugates. Carbohyd. Letters 1:69-275). Les glycopeptides sous la forme de dérivés phénylisothiocyanates sont fixés sur certaines fonctions ε-aminées des résidus lysyles libres de la polylysine comme précédemment décrit dans Midoux *et al*., (Nucleic Acids Res. 1993, 21:871-878).

C) Méthode V

polylysine gluconoylée et histidylée

a) les motifs monomères du polymère comportant une fonction NH3+ libre sont partiellement substitués par des résidus comportant une fonction permettant la fixation ultérieure d'autres molécules. S'agissant de la fixation de signaux de reconnaissance, par exemple un sel de polylysine (notamment sous forme de *p*-toluène sulfonate) est dissous dans un solvant organique (notamment le diméthylsulfoxyde) en présence d'une base (notamment la diisopropyléthylamine) et de l'ester N-hydroxysuccinimide de l'acide

dithiopyridine propionate ou de ses dérivés.

b) les motifs monomères du polymère comportant une fonction NH3+ encore libre sont ensuite partiellement substitués par des résidus non chargés entraînant une diminution de charge. S'agissant de la fixation des résidus entraînant la diminution de charge, par exemple un sel de polylysine (notamment sous forme de *p*-toluène sulfonate) est dissous dans un solvant organique (notamment le diméthylsulfoxyde) en présence d'une base (notamment la diisopropyléthylamine) et d'un acide organique hydroxylé activé (notamment la δ-gluconolactone).

c) les motifs monomères du polymère comportant une fonction NH3+ encore libre sont ensuite partiellement substitués par des résidus entraînant une déstabilisation des membranes cellulaires. S'agissant de la fixation de résidus entraînant une déstabilisation des membranes cellulaires, par exemple un sel de polylysine (notamment sous forme de *p*-toluène sulfonate) est dissous dans un solvant organique (notamment le diméthylsulfoxyde) en présence d'une base (notamment la diisopropyléthylamine), de molécules entraînant une déstabilisation des membranes cellulaires (notamment l'histidine dont le groupe αNH3+ et le groupe NH du noyau imidazole sont protégées par du tertiobutyloxycarbonyle) et d'un agent de couplage (notamment l'hexafluorophosphate de benzotriazolyl-N-oxytrisdimethylaminophosphonium). Après purification, les fonctions aminées protégées des résidus histidyle sont déprotégées.

d) Les signaux de reconnaissance sont liés aux groupements dithiopyridyles du polymère.

A titre d'exemple de fixation des signaux de reconnaissance sur la polylysine histidylée, on indique ci-après la fixation d'osides ou d'oligosides.

1) fixation d'osides.

Les osides simples dérivés en glycopeptides avec une fonction dithiopyridyle (*pyro*glutamyl-NH-$(CH2)_2$-S-S-pyridine) selon une méthode décrite dans *Monsigny et al*., (Brevet Français 9407738 (Monsigny, M., Sdiqui, N., Roche, A.C. and Mayer, R. 1994 Nouveaux dérives d'oligosides, leur procédé de préparation et leurs applications) et *Sdiqui et al*., (1995 New synthesis of glyco-amino acid conjugates. Carbohyd. Letters 1:269-275) sont réduits et fixés en milieu aqueux tamponné à pH neutre sur les fonctions dithiopyridyles du polymère.

2) fixation d'oligosides

Les oligosides complexes tels que les asialo-oligosides bi-, tri- ou tétraantennés ou Lewis dérivés en glycopeptides avec une fonction dithiopyridyle (*pyro*glutamyl-NH-$(CH2)_2$-S-S-pyridine) selon une méthode décrite dans *Monsigny et al*., (Brevet Français 9407738 (Monsigny, M., Sdiqui, N., Roche, A.C. and Mayer, R. 1994 Nouveaux dérives d'oligosides, leur procédé de préparation et leurs applications) et Sdiqui *et al*., (1995 New synthesis of glyco-amino acid conjugates. Carbohyd. Letters 1:269-275) sont réduits et fixés en milieu aqueux tamponné à pH neutre sur les fonctions dithiopyridyles du polymère.

polylysine gluconoylée et nicotinylée

a) les motifs monomères du polymère comportant une fonction NH3+ encore libre sont partiellement substitués par des résidus non chargés entraînant une diminution de charge. S'agissant de la fixation des résidus entraînant la diminution de charge, par exemple un sel de polylysine (notamment sous forme de *p*-toluène sulfonate) est dissous dans un solvant organique (notamment le diméthylsulfoxyde) en présence d'une base (notamment la diisopropyléthylamine) et d'un acide organique hydroxylé activé (notamment la δ-gluconolactone).

b) les motifs monomères du polymère comportant une fonction NH3+ libre sont ensuite partiellement substitués par des résidus entraînant une déstabilisation des membranes cellulaires. Par exemple la polylysine gluconoylée est dissoute dans un solvant organique (notamment le diméthylsulfoxyde) en présence d'une base (notamment la diisopropyléthylamine), de molécules entraînant une déstabilisation des membranes cellulaires (notamment l'acide nicotinique) et d'un agent de couplage (notamment l'hexafluorophosphate de benzotriazolyl-N-oxytrisdimethylaminophosphonium).

c) Les signaux de reconnaissance sont liés à certains groupements ε-aminés des résidus lysyles du polymère.

A titre d'exemple de fixation des signaux de reconnaissance sur la polylysine histidylée, on indique ci-après la fixation d'osides ou d'oligosides.

1) fixation d'osides.

Des osides simples sous la forme de dérivés phénylisothiocyanates sont fixés sur certaines fonctions

ε-aminées des résidus lysyles libres de la polylysine comme précédemment décrit dans Midoux *et al.*, (Nucleic Acids Res. 1993, 21: 871-878).

2) fixation d'oligosides.

Les oligosides complexes tels que les asialo-oligosides bi-, tri- ou tétraantennés ou Lewis sont obtenus sous forme de dérivés phénylisothiocyanates de glycopeptides selon une méthode décrite dans Monsigny *et al.*, (Brevet Français 9407738 (Monsigny, M., Sdiqui, N., Roche, A.C. and Mayer, R. 1994 Nouveaux dérives d'oligosides, leur procédé de préparation et leurs applications) et Sdiqui *et al.*, (1995 New synthesis of glyco-amino acid conjugates. Carbohyd. Letters 1:269-275). Les glycopeptides sous la forme de dérivés phénylisothiocyanates sont fixés sur certaines fonctions ε-aminées des résidus lysyles libres de la polylysine comme précédemment décrit dans Midoux *et al.*, (Nucleic Acids Res. 1993, 21:871-878).

*II) Les signaux de reconnaissance sont fixés sur certains motifs monomériques du polymère avant l'introduction des résidus entraînant une déstabilisation des membranes cellulaires de la façon suivante :*

A) Méthode II

polylysine nicotinylée

Ces substitutions suivent l'un quelconque des protocoles connus de l'homme de l'art.

a) A titre d'exemple de fixation des signaux de reconnaissance sur la polylysine (notamment sous forme de *p*-toluène sulfonate) dissoute dans un solvant organique (notamment le diméthylsulfoxyde) en présence- d'une base (notamment la diisopropyléthylamine), on indique ci-après la fixation d'osides ou d'oligosides.

1) fixation d'osides.

Des osides simples sous la forme de dérivés phénylisothiocyanates sont fixés sur certaines fonctions ε-aminées des résidus lysyles libres de la polylysine comme précédemment décrit dans Midoux *et al.*, (Nucleic Acids Res. 1993, 21: 871-878).

2) fixation d'oligosides.

Les oligosides complexes tels que les asialo-oligosides bi-, tri- ou tétraantennés ou Lewis sont obtenus sous forme de dérivés phénylisothiocyanates de glycopeptides selon une méthode décrite dans Monsigny *et al.*, (Brevet Français 9407738 (Monsigny, M., Sdiqui, N., Roche, A.C. and Mayer, R. 1994 Nouveaux dérives d'oligosides, leur procédé de préparation et leurs applications) et Sdiqui *et al.*, (1995 New synthesis of glyco-amino acid conjugates. Carbohyd. Letters 1, 269-275). Les glycopeptides sous la forme de dérivés phénylisothiocyanates sont fixés sur certaines fonctions ε-aminées des résidus lysyles libres de la polylysine comme précédemment décrit dans Midoux *et al.*, (Nucleic Acids Res. 1993, 21:871-878).

b) S'agissant de la fixation des résidus entraînant la déstabilisation des membranes, par exemple la polylysine substituée par des osides ou des oligosides est dissoute dans un solvant organique (notamment le diméthylsulfoxyde) en présence d'une base (notamment la diisopropyléthylamine), de molécules entraînant une déstabilisation des membranes cellulaires (notamment l'acide nicotinique) et d'un agent de couplage (notamment l'hexafluorophosphate de benzotriazolyl-N-oxytrisdimethylaminophosphonium).

B) Méthode VI

polylysine gluconoylée et nicotinylée

Ces substitutions suivent l'un quelconque des protocoles connus de l'homme de l'art.

a) les motifs monomères du polymère comportant une fonction $NH3+$ encore libre sont partiellement substitués par des résidus non chargés entraînant une diminution de charge. S'agissant de la fixation des résidus entraînant la diminution de charge, par exemple un sel de polylysine (notamment sous forme de *p*-toluène sulfonate) est dissous dans un solvant organique (notamment le diméthylsulfoxyde) en présence d'une base (notamment la diisopropyléthylamine) et d'un acide organique hydroxylé activé (notamment la δ-gluconolactone).

b) A titre d'exemple de fixation des signaux-de reconnaissance sur la polylysine gluconoylée dissoute dans un solvant organique (notamment le diméthylsulfoxyde) en présence d'une base (notamment la diisopropyléthylamine), on indique ci-après la fixation d'osides ou d'oligosides.

1) fixation d'osides.

Des osides simples sous la forme de dérivés phénylisothiocyanates sont fixés sur certaines fonctions ε-aminées des résidus lysyles libres de la polylysine comme précédemment décrit dans Midoux *et al*., (Nucleic Acids Res. 1993, 21: 871-878).

2) fixation d'oligosides.

Les oligosides complexes tels que les asialo-oligosides bi-, tri- ou tétraantennés ou Lewis sont obtenus sous forme de dérivés phénylisothiocyanates de glycopeptides selon une méthode décrite dans Monsigny *et al*., (Brevet Français 9407738 (Monsigny, M., Sdiqui, N., Roche, A.C. and Mayer, R. 1994 Nouveaux dérives d'oligosides, leur procédé de préparation et leurs applications) et Sdiqui *et al*., (1995 New synthesis of glyco-amino acid conjugates. Carbohyd. Letters 1:269-275). Les glycopeptides sous la forme de dérivés phénylisothiocyanates sont fixés sur certaines fonctions ε-aminées des résidus lysyles libres de la polylysine comme précédemment décrit dans Midoux *et al*., (Nucleic Acids Res. 1993, 21:871-878).

c) S'agissant de la fixation des résidus entraînant la déstabilisation des membranes, par exemple la polylysine substituée par des osides ou des oligosides est dissoute dans un solvant organique (notamment le diméthylsulfoxyde) en présence d'une base (notamment la diisopropyléthylamine), de molécules entraînant une déstabilisation des membranes cellulaires (notamment l'acide nicotinique) et d'un agent de couplage (notamment l'hexafluorophosphate de benzotriazolyl-N-oxytrisdimethylaminophosphonium).

*III) les signaux de reconnaissance sont fixés sur certains résidus déstabilisants*.

A) Méthode IX

polylysine histidylée

a) les motifs monomères du polymère comportant une fonction NH3+ libre sont partiellement substitués par des résidus entraînant une déstabilisation des membranes cellulaires. Par exemple, après réaction en milieu organique avec l'histidine dont le groupe NH3+ et le groupe NH du noyau imidazole sont protégées par du tertiobutyloxycarbonyle en présence d'un agent de couplage tel que l'hexafluorophosphate de benzotriazolyl-N-oxytrisdimethylaminophosphonium. Après réaction et purification, les fonctions aminées des résidus histidyle du polymère obtenu sont déprotégées.

b) Les signaux de reconnaissance sont liés à certains groupements NH3+ des résidus entraînant une déstabilisation des membranes.

A titre d'exemple de fixation des signaux de reconnaissance sur la polylysine histidylée, on indique ci-après la fixation d'oligosides.

Les oligosides complexes tels que les asialo-oligosides de types triantenné ou tétraantenné ou Lewis sont obtenus sous forme de dérivés phénylisothiocyanates de glycopeptides selon une méthode décrite dans Monsigny *et al*., (Brevet Français 9407738 (Monsigny, M., Sdiqui, N., Roche, A.C. and Mayer, R. 1994 Nouveaux dérives d'oligosides, leur procédé de préparation et leurs applications) et Sdiqui *et al*., (1995 New synthesis of glyco-amino acid conjugates. Carbohyd. Letters 1:269-275). Les glycopeptides sous la forme de dérivés phénylisothiocyanates sont fixés en milieu aqueux tamponné à pH neutre sur certaines fonctions NH2 des résidus histidyles. A ce pH, la fixation sur les NH3+ lysines est très faible, voire impossible.

B) Méthode XII

polylysine gluconoylée et histidylée

a) les motifs monomères du polymère comportant une fonction NH3+ libre sont partiellement substitués par des résidus non chargés entraînant une diminution de charge. S'agissant de la fixation des résidus entraînant la diminution de charge, par exemple un sel de polylysine (notamment sous forme de *p*-toluène sulfonate) est dissous dans un solvant organique (notamment le diméthylsulfoxyde) en présence d'une base (notamment la diisopropyléthylamine) et d'un acide organique hydroxylé activé (notamment la δ-gluconolactone).

b) les motifs monomères du polymère comportant une fonction NH3+ encore libre sont ensuite partiellement substitués par des résidus entraînant une déstabilisation des membranes cellulaires. S'agissant de la fixation de résidus entraînant une déstabilisation des membranes cellulaires, par exemple un sel de polylysine (notamment sous forme de *p*-toluène sulfonate) est dissous dans un solvant organique (notamment le diméthylsulfoxyde) en présence d'une base (notamment la diisopropyléthylamine), de molécules entraînant une déstabilisation des membranes cellulaires (notamment l'histidine dont le groupe

αNH3+ et le groupe NH du noyau imidazole sont protégées par du tertiobutyloxycarbonyle) et d'un agent de couplage (notamment l'hexafluorophosphate de benzotriazolyl-N-oxytrisdimethylaminophosphonium). Après purification, les fonctions aminées protégées des résidus histidyles sont déprotégées.

c) Les signaux de reconnaissance sont liés à certains groupements NH3+ des résidus entraînant une déstabilisation des membranes.

A titre d'exemple de fixation des signaux de reconnaissance sur la polylysine histidylée, on indique ci-après la fixation d'oligosides.

Les oligosides complexes tels que les asialo-oligosides bi-, tri- ou tétraantennés ou Lewis sont obtenus sous forme de dérivés phénylisothiocyanates de glycopeptides selon une méthode décrite dans Monsigny *et al.*, (Brevet Français 9407738 (Monsigny, M., Sdiqui, N., Roche, A.C. and Mayer, R. 1994 Nouveaux dérives d'oligosides, leur procédé de préparation et leurs applications) et Sdiqui *et al.*, (1995 New synthesis of glyco-amino acid conjugates. Carbohyd. Letters 1:269-275). Les glycopeptides sous la forme de dérivés phénylisothiocyanates sont fixés en milieu aqueux tamponné à pH neutre sur certaines fonctions aNH2 des résidus histidyles ; à ce pH, la fixation sur les NH3 + lysines est très faible, voire impossible.

C) Méthode IX

polylysine imidazolée

a) les motifs monomères du polymère comportant une fonction NH3+ libre sont partiellement substitués par des résidus entraînant une déstabilisation des membranes cellulaires. S'agissant de la fixation de résidus entraînant une déstabilisation des membranes cellulaires, par exemple un sel de polylysine (notamment sous forme de *p*-toluène sulfonate) est dissous dans un solvant organique (notamment le diméthylsulfoxyde) en présence d'une base (notamment la diisopropyléthylamine), de molécules entraînant une déstabilisation des membranes cellulaires (notamment le 4-carboxyméthyl-imidazole) et d'un agent de couplage (notamment l'hexafluorophosphate de benzotriazolyl-N-oxytrisdiméthylaminophosphonium).

b) Les signaux de reconnaissance sont liés sur certains noyaux imidazole des résidus entraînant une déstabilisation des membranes.

A titre d'exemple de fixation des signaux de reconnaissance sur la polylysine imidazolée, on indique ci-après la fixation de peptides ou des glycopeptides.

Les résidus imidazole peuvent être facilement alkylés en milieu neutre par des composés possédant un groupement activé tel que par exemple, l'iodoacétamide ou ses dérivés ; ceci est connu depuis les travaux de Korman S. et Clarke H.T. en 1956 (J. Biol. Chem. 113:133). L'alkylation des imidazoles par un dérivé de l'iodoacétamide s'effectue à pH voisin de la neutralité, 7,0 par exemple. A ce pH, les groupes ε-aminés de la lysine ne sont pas affectés, ils le seraient à pH beaucoup plus alcalin 9 ou au delà. Les signaux de reconnaissance de nature peptidique ou glycopeptidique (Monsigny *et al.*, Brevet Français 9407738. 1994 Nouveaux dérives d'oligosides, leur procédé de préparation et leurs applications et Sdiqui *et al.*, 1995 New synthesis of glyco-amino acid conjugates. Carbohyd. Letters 1:269-275) peuvent être facilement substitués par un groupement iodoacétamide. Les dérivés du type $ICH_2CONHR$ : I-$CH_2$-CO-NH-peptide ou I-$CH_2$-CO-NH-glycopeptide, sont fixés en milieu aqueux tamponné à pH neutre sur l'azote 3 et avec une efficacité moindre sur l'azote 1, conduisant à des dérivés *N*-carboxyméthyl stables. On peut également utiliser des dérivés du bromoacetamide qui sont également d'excellents réactifs pour alkyler des résidus imidazole (voir par exemple Henrikson *et al.*, 1965 J. Biol. Chem. 240:2921). Ce type de substitution, sous réserve de substituer un faible nombre de résidus imidazole par des signaux de reconnaissance ayant une haute affinité suffisante pour leur récepteur, ne fait pas perdre au polymère substitué par des résidus imidazole sa capacité déstabilisatrice des membranes à pH légèrement acide.

*IV) Les signaux de reconnaissance sont fixés sur certains résidus neutralisants*.

A) Méthode XIII

polylysine gluconoylée et imidazolée

a) les motifs monomères du polymère comportant une fonction NH3+ libre sont partiellement substitués par des résidus non chargés entraînant une diminution de charge. S'agissant de la fixation des résidus entraînant la diminution de charge, par exemple un sel de polylysine (notamment sous forme de p-toluène sulfonate) est dissous dans un solvant organique (notamment le diméthylsulfoxyde) en présence d'une base (notamment la diisopropyléthylamine) et par deux acides organiques hydroxylés activés (notamment la 6-désoxy-6-iodo-δ-gluconolactone pour une part et la δ-gluconolactone pour 10 à 50 parts).

b) les motifs monomères du polymère comportant une fonction NH3+ libre sont partiellement substitués par des résidus entraînant une déstabilisation des membranes cellulaires. S'agissant de la fixation de

résidus entraînant une déstabilisation des membranes cellulaires, par exemple un sel de polylysine (notamment sous forme de *p*-toluène sulfonate) est dissous dans un solvant organique (notamment le diméthylsulfoxyde) en présence d'une base (notamment la diisopropyléthylamine), de molécules entraînant une déstabilisation des membranes cellulaires (notamment le 4-carboxyméthyl-imidazole) et d'un agent de couplage (notamment l'hexafluorophosphate de benzotriazolyl-N-oxytrisdiméthylaminophosphonium).
c) Les signaux de reconnaissance sont liés à certains des résidus neutralisants.

A titre d'exemple de fixation des signaux de reconnaissance sur la polylysine gluconoylée et histidylée, on indique ci-après la fixation d'oligosides.

Les oligosides complexes tels que les asialo-oligosides bi-, tri- ou tétraantennés ou Lewis dérivés en glycopeptides avec une fonction dithiopyridyle (*pyro*glutamyl-NH-$(CH2)_2$-S-S-pyridine) selon une méthode décrite dans Monsigny *et al*., (Brevet Français 9407738 (Monsigny, M., Sdiqui, N., Roche, A.C. and Mayer, R. 1994 Nouveaux dérives d'oligosides, leur procédé de préparation et leurs applications) et Sdiqui *et al*., (1995 New synthesis of glyco-amino acid conjugates. Carbohyd. Letters 1:269-275) sont réduits par le triscarboxyéthylphosphine en milieu neutre (pH voisin de 7,0) par exemple et fixés en milieu aqueux tamponné à pH légèrement alcalin (aux environs de pH 8,5) sur les résidus 6-désoxy-6-iodo-gluconoyles du polymère. Ce type de substitution, sous réserve de substituer un faible nombre de résidus imidazole par des signaux de reconnaissance ayant une haute affinité pour leur récepteur, ne fait pas perdre au polymère substitué par des résidus imidazole sa capacité détabilisatrice des membranes à pH légèrement acide.

B) Méthode XIII

polylysine gluconoylée et imidazolée

a) les motifs monomères du polymère comportant une fonction NH3+ libre sont partiellement substitués par des résidus non chargés entraînant une diminution de charge. S'agissant de la fixation des résidus entraînant la diminution de charge, par exemple un sel de polylysine (notamment sous forme de *p*-toluène sulfonate) est dissous dans un solvant organique (notamment le diméthylsulfoxyde) en présence d'une base (notamment la diisopropyléthylamine) et par deux acides organiques hydroxylés activés (notamment la 6-bromoacétamido-L-gulono-1,5 lactone pour une part et la δ-gluconolactone pour 10 à 50 parts). La 6-bromoacétamido-L-gulono-1,5 lactone est obtenue après réduction par le cyanoborohydrure de l'imine obtenue en mélangeant une solution ammoniacale ($NH_4OH$ ou (NH4)2CO3) et une solution d'acide uronique, l'acide glucuronique par exemple, puis par acylation de l'amine par un bromoacétate activé, par exemple l'anhydride bromoacétique ou le bromo acétate de succinimidyle.
b) les motifs monomères du polymère comportant une fonction NH3+ libre sont partiellement substitués par des résidus entraînant une déstabilisation des membranes cellulaires. S'agissant de la fixation de résidus entraînant une déstabilisation des membranes cellulaires, par exemple un sel de polylysine (notamment sous forme de *p*-toluène sulfonate) est dissous dans un solvant organique (notamment le diméthylsulfoxyde) en présence d'une base (notamment la diisopropyléthylamine), de molécules entraînant une déstabilisation des membranes cellulaires (notamment le 4-carboxyméthyl-imidazole) et d'un agent de couplage (notamment l'hexafluorophosphate de benzotriazolyl-N-oxytrisdiméthylaminophosphonium).
c) Les signaux de reconnaissance sont liés à certains des résidus neutralisants.

A titre d'exemple de fixation des signaux de reconnaissance sur la polylysine gluconoylée et histidylée, on indique ci-après la fixation d'oligosides.

Les oligosides complexes tels que les asialo-oligosides bi-, tri- ou tétraantennés ou Lewis dérivés en glycopeptides avec une fonction dithiopyridyle (*pyro*glutamyl-NH-$(CH2)_2$-S-S-pyridine) selon une méthode décrite dans Monsigny *et al*., (Brevet Français 9407738 (Monsigny, M., Sdiqui, N., Roche, A.C. and Mayer, R. 1994 Nouveaux dérives d'oligosides, leur procédé de préparation et leurs applications) et Sdiqui *et al*., (1995 New synthesis of glyco-amino acid conjugates. Carbohyd. Letters 1:269-275) sont réduits par le triscarboxyéthylphosphine en milieu neutre (pH voisin de 7,0) par exemple et fixés en milieu aqueux tamponné à pH légèrement alcalin (aux environs de pH 8,5) sur les résidus bromoacétamido gulonyle du polymère. Ce type de substitution, sous réserve de substituer un faible nombre de résidus imidazole par des signaux de reconnaissance ayant une haute affinité pour leur récepteur, ne fait pas perdre au polymère substitué par des résidus imidazole sa capacité déstabilisatrice des membranes à pH légèrement acide.

Le complexe acide nucléique/conjugué polymérique est obtenu en mélangeant une solution de l'acide nucléique concerné et une solution du conjugué polymérique. De préférence, lesdites solutions sont préparées à partir du sérum physiologique ou d'un tampon ou d'un milieu cytocompatible.

Selon un mode de réalisation avantageux de l'invention, on utilise un complexe tel que décrit ci-dessus ou un conjugué tel que décrit ci-dessus pour la transfection *in vitro*, *ex vivo* ou *in vivo* de cellules à l'aide d'un gène,

notamment ceux définis précédemment.

Selon un mode de réalisation avantageux de l'invention, on utilise un complexe ou un conjugué tels que décrits ci-dessus, se caractérisant en ce que les cellules sont choisies parmi:

- des cellules souches hématopoiétiques;
- des cellules dendritiques;
- cellules du foie;
- cellules des muscles squelettiques;
- cellules de la peau:
    - . fibroblastes,
    - . kératinocytes,
    - . cellules dendritiques,
    - . mélanocytes.
- cellules des parois vasculaires;
    - . endothéliales;
    - . musculaires lisses;
- cellules épithéliales des voies aériennes;
- cellules du système nerveux central;
- cellules cancéreuses;
- cellules du système immunitaire, telles que des lymphocytes, des macrophages, des cellules NK etc...

Selon un mode de réalisation avantageux de l'invention, la méthode de transfection *in vitro* ou *ex vivo*, se caractérise en ce que l'on met en présence un complexe tel que décrit précédemment dans un milieu contenant des cellules à transfecter, dans des conditions telles qu'il y a:

- passage du complexe à partir du milieu dans le cytoplasme des cellules,
- relargage de l'acide nucléique impliqué dans le susdit complexe dans le cytosol et/ou le noyau des cellules,
- transcription et expression de l'acide nucléique dans les cellules transfectées,
- expression de la protéine correspondant au gène transfecté.

L'invention concerne également une composition pharmaceutique, qui se caractérise en ce qu'elle comprend à titre de substance active, l'un au moins des complexes tels que décrits ci-dessus, ou l'un au moins des conjugués tels que décrits ci-dessus, en association avec un véhicule pharmaceutiquement acceptable.

Selon un mode de réalisation avantageux de l'invention, on utilise un complexe tel que décrit ci-dessus ou un conjugué tel que décrit ci-dessus pour la préparation d'un médicament destiné par exemple au traitement de déficience métabolique congénitale ou acquise, ou au traitement de tumeurs, ou pour la préparation d'un vaccin, par exemple vaccin contre la grippe.

L'invention a également pour objet une trousse ou un kit comprenant:

- un conjugué polymérique tel que décrit ci-dessus, tel que la polylysine, substituée par un résidu entraînant en milieu faiblement acide une déstabilisation des membranes cellulaires, ce conjugué polymérique étant apte à comporter éventuellement un signal de reconnaissance, lequel est préalablement fixé ou non sur le susdit conjugué polymérique, ledit signal de reconnaissance étant fonction de la cellule à cibler,
- éventuellement un plasmide contenant au moins un gène à transférer, et éventuellement le système de régulation de l'expression du susdit gène,
- des réactifs permettant la fixation éventuelle du signal de reconnaissance sur le susdit conjugué polymérique,
- des réactifs permettant la formation d'un complexe tel que décrit ci-dessus, ou entre le conjugué polymérique et le gène à transférer, ou entre le conjugué polymérique et un plasmide contenant le gène à transférer,
- des réactifs permettant la transfection de la cellule par le susdit complexe.

## DESCRIPTION DES FIGURES :

Figure 1

Elle représente un fragment de polylysine (DP 190) partiellement substituée par des résidus histidyle.

Figure 2

Elle représente le spectre RMN à 300 MHz dans $D_2O$ de la polylysine (DP 190) substituée par 70 résidus histidyle :

1,28 à 1,88 ppm : 6 protons des carbones 3, 4 et 5 des lysines substituées ou non substituées.
2,39 ppm : protons du groupe $CH_3$ du *p*-toluène sulfonate
2,75 ppm : trace de DMSO
2,99 ppm : 2 protons du carbone 6 d'un résidu lysyle non substitué
3,15 ppm : 2 protons du carbone 6 d'un résidu lysyle substitué
3,35 ppm : 2 protons du carbone 9 d'un résidu histidyle
4,36 ppm : 2 protons des carbones 2 et 8
4,78 ppm : pic de l'eau
7,36 ppm : 2 protons (doublet, constante de couplage en ortho = 7,97 Hz) des protons des carbones 2 et 6 du cycle aromatique du *p*-toluène sulfonate
7,42 ppm : 1 proton du carbone 11 d'un résidu histidyle
7,71 ppm : 2 protons (doublet, constante de couplage en ortho = 8,01 Hz) des protons des carbones 3 et 5 du cycle aromatique du *p*-toluène sulfonate
8,7 ppm : 1 proton du carbone 12 d'un résidu histidyle.

Figure 3

Elle concerne la préparation de la polylysine (DP 190) partiellement substituée par 70 résidus histidyle.

La poly-L-lysine sous forme bromhydrate (masse moléculaire moyenne 40 000 ; degré de polymérisation moyen 190) (1 g dans 200 ml $H_2O$) provenant de chez Bachem Feinchemikalien (Budendorf, Suisse) est d'abord passée sur une colonne échangeuse d'anions (Dowex 2 x 8, forme OH- ; 35 x 2,5 cm) dans le but d'enlever le bromure qui est toxique pour les cellules. La solution de polylysine est neutralisée avec une solution d'acide *p*-toluène sulfonique à 10% dans l'eau puis lyophilisée.

La polylysine est partiellement substituée avec des résidus histidyle comme suit : la polylysine sous forme *p*-toluène sulfonate (50 mg ; 0,96 μmoles) dissoute dans 3 ml de DMSO (diméthylsulfoxide) en présence de diisopropyléthylamine (42 μl ; 288 μmoles), est mise à réagir pendant 24 heures à 20°C avec 32 mg de (Boc)His(Boc) -OH (96 μmoles) en présence de 43 mg d'hexafluorophosphate de benzotriazolyl N-oxytrisdiméthylaminophosphonium (BOP) (97 μmoles). Les résidus histidyle sont ensuite déprotégés en présence de 20 ml d'un mélange eau et acide trifluoroacétique (TFA) (50/50 V/V) pendant 24 heures à 20°C. L'eau et le TFA sont éliminés par évaporation sous pression réduite. Le polymère est précipité en ajoutant 10 volumes d'isopropanol. Après centrifugation (1800 g x 15 minutes), le culot est lavé avec de l'isopropanol et récupéré après une nouvelle centrifugation. Le culot est repris dans l'eau distillée et la solution est lyophilisée. Le nombre x de résidus histidyle fixés par molécule de polylysine est déterminé par RMN du proton comme suit :

$$x = 6(h_{8.7} / h_{Lys})DP$$

où $h_{8.7}$ est l'intégrale du pic à 8,7 ppm correspondant au proton du carbone 12 d'un résidu histidyle, $h_{Lys}$ est l'intégrale des pics entre 1,28 et 1,88 ppm correspondant aux 6 protons des carbones 3, 4 et 5 des résidus lysine et DP est le degré de polymérisation de la polylysine (DP = 190). Le nombre de résidus histidyle fixés par molécule de polylysine est x, x = 70 dans la préparation décrite ci-dessus.

Figure 4

Elle représente le transfert de gènes dans les cellules HepG2 en utilisant la polylysine (DP 190) partiellement substituée par des résidus histidyle (HispLK).

Les complexes ADN/HispLK sont formés en mélangeant le plasmide pCMVLUC (10 μg dans 0, 7 ml de DMEM) et la polylysine substituée par 70 résidus histidyle (40 μg dans 0, 3 ml de DMEM). Après 30 minutes à 20°C, la solution contenant les complexes est diluée une fois avec du DMEM et complétée avec 5% de sérum bovin foetal.

Les complexes ADN/pLK sont formés en mélangeant le plasmide pCMVLUC (10 µg dans 0, 7 ml de DMEM) et la polylysine (5 µg dans 0, 3 ml de DMEM). Après 30 minutes à 20°C, la solution contenant les complexes est diluée une fois avec du DMEM et complétée avec 5% de sérum bovin foetal et éventuellement avec 100 µM de chloroquine (+ chloro) ou 20 µM d'un peptide fusiogène (+ E5CA) (GLFEAIAEFIEGGWEGLIEGCA). Le milieu dans lequel les cellules HepG2 ($3 \times 10^5$ cellules/4 $cm^2$) ont poussé pendant 24 heures, est éliminé et remplacé par une solution (1 ml) contenant un complexe ADN/polymère (5 µg/ml d'ADN). Après 4 heures d'incubation à 37°C, le milieu, des cellules est de nouveau éliminé et les cellules sont incubées dans du milieu de culture en présence de 10% de sérum bovin foetal. L'expression du gène de la luciférase a été déterminée 48 heures après la transfection, en mesurant, pendant 4 secondes la luminescence émise (RLU : valeurs relatives de la lumière émise exprimées en unités arbitraires) dans les lysats cellulaires.

Dans ces conditions, 1 pg/ml de luciférase produit 2000 RLU.

En allant de gauche à droite sur l'axe des abscisses, le premier rectangle correspond au complexe ADN/polylysine histidylée, le deuxième rectangle correspond au complexe ADN/polylysine, le troisième rectangle correspond au complexe ADN/polylysine additionné de chloroquine, le quatrième rectangle correspond au complexe ADN/polylysine additionné du peptide fusiogène E5CA.

En encadré : variation de l'efficacité de transfection en fonction de la quantité de plasmide.

Figure 5

Elle concerne le transfert de gènes dans les cellules HepG2 en utilisant la polylysine (DP 190) partiellement substituée par des résidus histidyle. Elle représente l'influence du nombre de résidus histidyle fixé par molécule de polylysine sur l'efficacité de la transfection.

En allant de gauche à droite sur l'axe des abscisses, le premier rectangle correspond au complexe ADN/polylysine non substitué, le deuxième rectangle correspond au complexe ADN/polylysine substitué par 19 résidus d'histidyle, le troisième rectangle correspond au complexe ADN/polylysine substitué par 30 résidus d'histidyle, le quatrième rectangle correspond au complexe ADN/polylysine substitué par 46 résidus d'histidyle, le cinquième rectangle correspond au complexe ADN/polylysine substitué par 63 résidus d'histidyle, le sixième rectangle correspond au complexe ADN/polylysine substitué par 70 résidus d'histidyle et le septième rectangle correspond au complexe ADN/polylysine substitué par 84 résidus d'histidyle.

Les complexes ADN/HispLK sont formés en mélangeant le plasmide pCMVLUC (10 µg dans 0, 7 ml de DMEM) et la polylysine substituée par un nombre variable de résidus histidyle (40 µg dans 0, 3 ml de DMEM). Après 30 minutes à 20°C, la solution contenant les complexes est diluée une fois avec du DMEM et complétée avec du sérum bovin foetal (concentration finale 10%). Le milieu dans lequel les cellules HepG2 ($3 \times 10^5$ cellules/4 $cm^2$) ont poussé pendant 24 heures, est éliminé et remplacé par une solution (1 ml) contenant un complexe ADN/polymère (5 µg/ml d'ADN). Après 4 heures d'incubation à 37°C, le milieu des cellules est de nouveau éliminé et les cellules sont incubées dans du milieu de culture en présence de 10% de sérum bovin foetal. L'expression du gène de la luciférase a été déterminée 48 heures après la transfection, en mesurant, pendant 4 secondes, la luminescence émise (RLU : valeurs relatives de la lumière émise exprimées en unités arbitraires) dans les lysats cellulaires.

Dans ces conditions, 1 pg/ml de luciférase produit 2000 RLU.

Figure 6

Elle concerne le transfert de gènes dans les cellules HOS en utilisant la polylysine (DP 190) partiellement substituée par des résidus histidyle. Elle représente l'influence sur l'efficacité de la transfection du rapport ADN/polymère (exprimé sur les abscisses en µg de polymère pour 10 µg d'ADN) dans les complexes pCMVLUC/$His_{84}$pLK.

Les complexes ADN/HispLK sont formés en mélangeant le plasmide pCMVLUC (10 µg dans 0,7 ml de DMEM) et différentes quantité de polylysine substituée par 84 résidus histidyle dans 0, 3 ml de DMEM. Après 30 minutes à 20°C, la solution contenant les complexes est diluée une fois avec du DMEM et complétée avec 1% de sérum bovin foetal. Le milieu dans lequel les cellules HOS ($2 \times 10^5$ cellules/4 $cm^2$) ont poussé pendant 24 heures est éliminé et remplacé par une solution (1 ml) contenant un complexe ADN/polymère (5 µg/ml d'ADN). Après 4 heures d'incubation à 37°C, le milieu des cellules est de nouveau éliminé et les cellules sont incubées dans du milieu de culture en présence de 10% de sérum bovin foetal. L'expression du gène de la luciférase a été déterminée 48 heures après la transfection, en mesurant pendant 4 secondes la luminescence émise (RLU : valeurs relatives de la lumière émise exprimées en unités arbitraires) dans les lysats cellulaires.

Dans ces conditions, 1 pg/ml de luciférase produit 2000 RLU.

Figure 7

Elle concerne le transfert de gènes dans les cellules HepG2 en utilisant la polylysine (DP 190) partiellement

substituée par des résidus histidyle. Elle représente l'influence du temps d'incubation (exprimé en heures sur les abscisses) des complexes pCMVLUC/$His_{70}$pLK avec les cellules sur l'efficacité de la transfection. Les complexes ADN/HispLK sont formés en mélangeant le plasmide pCMVLUC (10 µg dans 0, 7 ml de DMEM) et la polylysine substituée par 70 résidus histidyle (40 µg dans 0, 3 ml de DMEM). Après 30 minutes à 20°C, la solution contenant les complexes est diluée une fois avec du DMEM et complétée à 10% avec du sérum bovin foetal. Le milieu dans lequel les cellules HepG2 (3 x $10^5$ cellules/4 $cm^2$) ont poussé pendant 24 heures est éliminé et remplacé par une solution (1 ml) contenant un complexe ADN/polymère (5 µg/ml d'ADN). Après les différents temps d'incubation à 37°C, le milieu des cellules est de nouveau éliminé et les cellules sont incubées dans du milieu de culture en présence de 10% de sérum bovin foetal. L'expression du gène de la luciférase a été déterminée 48 heures après la transfection, en mesurant, pendant 4 secondes la luminescence émise (RLU : valeurs relatives de la lumière émise exprimées en unités arbitraires) dans les lysats cellulaires.

Dans ces conditions, 1 pg/ml de luciférase produit 2000 RLU.

Figure 8

Elle concerne le transfert de gènes dans les cellules HepG2 en utilisant la polylysine (DP 190) partiellement substituée par des résidus histidyle. Elle représente l'influence sur l'efficacité de la transfection de la quantité de sérum bovin foetal (exprimé sur l'axe des abscisses en % de sérum dans le milieu utilisé) présent pendant l'incubation des complexes pCMVLUC/$His_{70}$pLK avec les cellules. Les complexes ADN/HispLK sont formés en mélangeant le plasmide pCMVLUC (10 µg dans 0, 7 ml de DMEM) et la polylysine substituée par 70 résidus histidyle (40 µg dans 0, 3 ml de DMEM). Après 30 minutes à 20°C, la solution contenant les complexes est diluée une fois avec du DMEM et complétée avec différentes quantité de sérum bovin foetal. Le milieu dans lequel les cellules HepG2 (3 x $10^5$ cellules/4 $cm^2$) ont poussé pendant 24 heures, est éliminé et remplacé par une solution (1 ml) contenant un complexe ADN/polymère (5 µg/ml d'ADN). Après 4 heures d'incubation à 37°C, le milieu des cellules est de nouveau éliminé et les cellules sont incubées dans du milieu de culture en présence de 10% de sérum bovin foetal. L'expression du gène de la luciférase a été déterminée 48 heures après la transfection, en mesurant, pendant 4 secondes la luminescence émise (RLU : valeurs relatives de la lumière émise exprimées en unités arbitraires) dans les lysats cellulaires.

Dans ces conditions, 1 pg/ml de luciférase produit 2000 RLU.

Figure 9

Elle concerne le transfert de gènes dans différentes lignées cellulaires en utilisant la polylysiné (DP 190) substituée par 84 résidus histidyle. Les complexes ADN/HispLK sont formés en mélangeant le plasmide pCMVLUC (10 µg dans 0, 7 ml de DMEM) et de la polylysine substituée par 84 résidus histidyle dans 0, 3 ml de DMEM. Après 30 minutes à 20°C, la solution contenant les complexes est diluée une fois avec du DMEM et complétée à 10% avec du sérum bovin foetal. Le milieu dans lequel les cellules (2-3 x $10^5$ cellules/4 $cm^2$) ont poussé pendant 24 heures, est éliminé et remplacé par une solution contenant un complexe ADN/polymère (5 µg/ml d'ADN). Après 4 heures d'incubation à 37°C, le milieu des cellules est de nouveau éliminé et les cellules sont incubées dans du milieu de culture en présence de 10% de sérum bovin foetal. L'expression du gène de la luciférase a été déterminée 48 heures après la transfection, en mesurant, pendant 4 secondes la luminescence émise (RLU : valeurs relatives de la lumière émise exprimées en unités arbitraires) dans les lysats cellulaires. HepG2 = lignée de cellules dérivant d'un hépatocarcinome humain ; HOS = lignée de cellules dérivant d'un ostéosarcome humain; MCF-7 ; lignée de cellules dérivant d'un adénocarcinome humain ; B 16 = lignée de cellules dérivant d'un mélanome murin ; COS = lignée de cellules dérivant de cellules de reins de singe transformées par SV40 ; Rb1 = lignée de cellules dérivant de cellules musculaires lisses d'aorte de lapin ; HeLa = lignée de cellules épithéloïdes humaines ; 16 HBE = lignée de cellules épithéliales des voies respiratoires humaines normales ; ΣCFTE = lignée de cellules épithéliales des voies respiratoires humaines déficientes pour le gène responsable de la mucoviscidose (CFTR).

**Préparation de la polylysine histidylée substituée par le lactose**

*- préparation de la polylysine substituée par des goupements thiol activés*

La polylysine sous forme hydrobromide (masse moléculaire moyenne 40 000 ; degré de polymérisation moyen 190) (1 g dans 200 ml $H_2O$) provenant de chez Bachem Feinchemikalien (Budendorf, Suisse) est d'abord passée sur une colonne échangeuse d'anions (Dowex 2 x 8, forme OH- ; 35 x 2,5 cm) dans le but d'enlever le bromure qui est toxique pour les cellules. La solution de polylysine est neutralisée avec une solution d'acide *p*-toluène sulfonique à 10% dans l'eau puis lyophilisée.

La polylysine *p*-toluène sulfonate (50 mg ; 0,91 µmol) est dissoute dans 2 ml de DMSO et mise à réagir à 20°C pendant 12 h avec l'ester N-hydroxysuccinimide du 4-carbonyl-α-methyl-α-(2-pyridinyldithio) toluène (SMPT,

Pierce, USA) (5,3 mg ; 13,6 μmol). La polylysine substituée par des groupes carbonyl α-méthyl-α-(2-pyridinyldithio) toluène (=MPT-pLK) est précipité en ajoutant 10 volumes d'isopropanol. Après centrifugation (1800 g × 15 minutes), le culot est lavé avec de l'isopropanol et récupéré après une nouvelle centrifugation. Le culot est repris dans l'eau distillée et la solution est lyophilisée. Le nombre moyen de molécules de MPT liées par molécule de polylysine est déterminé par absorbance à 343 nm de la pyridine thione (ε = 8080 $M^{-1}$ x $cm^{-1}$) libérée par réduction quantitative de la liaison disulfure à l'aide de (TCEP : tris-carboxyéthylphosphine) : le nombre moyen de MPT par molécule de polylysine est 10.

*- préparation de la polylysine histidylée substituée par des goupements thiol activés*.

La polylysine sous forme *p*-toluène sulfonate substituée par 10 résidus MPT (50 mg ; 0,96 μmoles) dissoute dans 3 ml de DMSO (dimethylsulfoxide) en présence de diisopropyléthylamine (42 μl ; 288 μmoles), est mise à réagir pendant 24 heures à 20°C avec 32 mg de (Boc)His(Boc)-OH (80 μmoles) en présence de 43 mg d'hexafluorophosphate de benzotriazolyl N-oxytrisdiméthylaminophosphonium (BOP) (97 μmoles). Les résidus histidyle sont ensuite déprotégés en présence de 20 ml d'un mélange eau et acide trifluoroacétique (TFA)(50/50 V/V) à 50% pendant 24 heures à 20°C. L'eau et le TFA sont éliminés par évaporation sous pression réduite. Le polymère est précipité en ajoutant 10 volumes d'isopropanol. Après centrifugation (1800 g × 15 minutes), le culot est lavé avec de l'isopropanol et récupéré après une nouvelle centrifugation. Le culot est repris dans l'eau distillée et la solution est lyophilisée. Le nombre de résidus histidyle fixés par molécule de polylysine, déterminé par RMN du proton est 60.

*- réduction du dithiopyridyle*

L'oligoside est d'abord transformé en glycopeptide selon une méthode décrite dans la demande de Brevet Français 9407738 (Monsigny, M., Sdiqui, N., Roche, A.C. and Mayer, R. (1994) Nouveaux dérivés d'oligosides, leur procédé de préparation et leurs applications).

Le glycopeptide est fixé sur la polylysine partiellement histidylée via un pont disulfure.

Le Galβ4Glcβ-pyroglutamyl-NH-$(CH2)_2$-S-S-pyridine (3 μmol) est traité par 3,5 μmole de TCEP (tris-carboxyéthylphosphine) dans un tampon phosphate de sodium 0,1 M à pH 7 (1 ml), pendant 1 h à 20°C. Cette solution est ajoutée à la polylysine partiellement histidylée substituée par 10 résidus MPT (10 mg ; 0,2 μmoles) dissoute dans le tampon phosphate de sodium 0,1 M à pH 7 (1 ml). Après 1 h à 20°C, le polymère est précipité par addition de 10 volumes d'isopropanol. Le précipité est récupéré après centrifugation (1 800 g, 15 min) et lavé dans l'isopropanol puis dissous dans l'eau et lyophilisé.

Le rendement de la réaction de couplage dans les conditions utilisées est égal ou supérieur à 90%.

**Préparation de la polylysine histidylée et substituée par un oligoside complexe : le Lewis$^b$**

Exemple du Lewis$^b$ = Fucα4(Fucα2Galβ3)GlcNAcβ3Galβ4Glc

Les oligosides complexes ayant un résidu glucose (Glc) ou N-acétyl glucosamine (GlcNAc) en position réductrice sont d'abord transformés en glycopeptides selon une méthode décrite dans la demande de Brevet Français 9407738 (Monsigny, M., Sdiqui, N., Roche, A.C. and Mayer, R. (1994) Nouveaux dérivés d'oligosides, leur procédé de préparation et leurs applications).

Les oligosides complexes sont fixés sur la polylysine partiellement histidylée selon une liaison du glycopeptide à la polylysine histidylée via un pont disulfure.

L'oligoside Fucα4(Fucα2Galβ3)GlcNAcβ3Galβ4Glc est dérivé en glycopeptide Fucα4(Fucα2Galβ3) GlcNAcβ3Galβ4Glcβ-pyroglutamyl-R. Le groupe carboxylique du pyroglutamyle est substitué par une fonction dithiopyridine pour donner le glycopeptide : Fucα4(Fucα2Galβ3)GlcNAcβ3Gal β4Glcβ-pyroglutamyl-NH-$(CH2)_2$-S-S-pyridine (demande de Brevet Français 9407738: Monsigny, M., Sdiqui, N., Roche, A.C. and Mayer, R. (1994) Nouveaux dérives d'oligosides, leur procédé de préparation et leurs applications et Quétard *et al.*, Simple synthesis of novel glycosynthons for glycoconjugate préparation : oligosylpyroglutamyl derivatives, en préparation).

*- réduction du glycopeptide*

Le glycopeptide (2 μmol) est traité par 2,2 μmole de TCEP (tris-carboxyéthylphosphine) dans un tampon phosphate de sodium 0,1 M à pH 7 (1 ml), pendant 1 h à 20°C. Cette solution est ajoutée à la polylysine partiellement histidylée substituée par 10 résidus MPT (10 mg; 0,2 μmoles) dissoute dans le tampon phosphate de sodium 0,1 M à pH 7 (1 ml). Après 1 h à 20°C, le polymère est précipité par addition de 10 volumes d'isopropanol. Le précipité est récupéré après centrifugation (1 800 g, 15 min) et lavé dans l'isopropanol puis dissous dans l'eau et lyophilisé.

Le rendement de la réaction de couplage dans les conditions utilisées est égal ou supérieur à 90%.

**Préparation de la polylysine histidylée substituée par le peptide ANP**

*- préparation de la polylysine substituée par des goupements thiol activés*

La polylysine sous forme bromhydrate (masse moléculaire moyenne 40 000 ; degré de polymérisation moyen 190) (1 g dans 200 ml $H_2O$) provenant de chez Bachem Feinchemikalien (Budendorf, Suisse) est d'abord passée sur une colonne échangeuse d'anions (Dowex 2 x 8, forme OH- ; 35 x 2,5 cm) dans le but d'enlever le bromure qui est toxique pour les cellules. La solution de polylysine est neutralisée avec une solution d'acide *p*-toluène sulfonique à 10% dans l'eau puis lyophilisée.

La polylysine *p*-toluène sulfonate (50 mg; 0,91 μmol) est dissoute dans 2 ml de DMSO et mise à réagir à 20°C pendant 12 h avec l'ester N-hydroxysuccinimide du 4-carbonyl-α-methyl-α-(2-pyridinyldithio) toluène (SMPT, Pierce, USA) (5,3 mg ; 13,6 μmol). Le polymère (MPT-pLK) est précipité en ajoutant 10 volumes d'isopropanol. Après centrifugation (1800 g × 15 minutes), le culot est lavé avec de l'isopropanol et récupéré après une nouvelle centrifugation. Le culot est repris dans l'eau distillée et la solution est lyophilisée. Le nombre moyen de molécules de MPT liées par molécule de polylysine est déterminé par absorbance à 343 nm de la pyridine thione ($\varepsilon$= 8080 $M^{-1}$ x $cm^{-1}$) libérée par réduction quantitative de la liaison disulfure à l'aide de (TCEP) : le nombre moyen de MPT est 10.

*- préparation de la polylysine histidylée substituée par des goupements thiol activés*.

La polylysine sous forme *p*-toluène sulfonate substituée par 10 résidus MPT (50 mg ; 0,96 μmoles) dissoute dans 3 ml de DMSO (diméthylsulfoxide) en présence de diisopropyléthylamine (42 μl ; 288 μmoles), est mise à réagir pendant 24 heures à 20°C avec 32 mg de (Boc)His(Boc)-OH (80 μmoles) en présence de 43 mg d'hexafluorophosphate de benzotriazolyl N-oxytrisdiméthylaminophosphonium (BOP) (97 μmoles). Les résidus histidyle sont ensuite déprotégés en présence de 20 ml d'une solution d'acide trifluoroacétique (TFA) à 50% pendant 48 heures à 20°C. L'eau et le TFA sont éliminés par évaporation sous pression réduite. Le polymère est précipité en ajoutant 10 volumes d'isopropanol. Après centrifugation (1800 g × 15 minutes), le culot est lavé avec de l'isopropanol et récupéré après une nouvelle centrifugation. Le culot est repris dans l'eau distillée et la solution est lyophilisée. Le nombre x de résidus histidyle fixés par molécule de polylysine, déterminé par RMN du proton est 60.

*- réduction du peptide ANP*

Le peptide ANP (CYSLRRSSAFGGRIDRIGAQSA) ayant sa cystéine en position N-terminale protégée sous forme thiopyridinyle (7,5 mg ; 2 μmol) est mis à réagir à 20°C pendant 15 minutes avec du TCEP (0,7 mg ; 2 μmol) dans 1 ml de tampon 0,1 M NaCl, 0,1 M tris/HCl pH 7,6.

*- préparation de la polylysine histidylée substituée avec le peptide ANP*

La polylysine partiellement histidylée substituée par 10 molécules de MPT ($MPT_{10}$-,$His_{70}$pLK) (10 mg; 0,2 μmol) dans 1 ml de tampon 0,1 M NaCl, 0,1 M tris/HCl pH 7,6 est mise à réagir à 20°C pendant 24 heures avec 7,5 mg (2 μmol) de peptide ANP dont la cystéine a été réduite. Le polymère (ANP-*S*-, $His_{70}$-pLK) est précipité en ajoutant 10 volumes d'isopropanol. Après centrifugation (1800 g × 15 minutes), le culot est lavé avec de l'isopropanol et récupéré après une nouvelle centrifugation. Le culot est repris dans l'eau distillée et la solution est lyophilisée. Le nombre moyen de molécules de peptide ANP fixées par molécule de polymère est déterminé par l'analyse des acides aminés du polymère par chromatographie à haute pression (HPLC) avec une colonne $C_{18}$ (Supelcosil LC-18-DB, Supelco, Bellefonte, PA, USA) en phase inverse, après hydrolyse du polymère dans HC1 5,6 N à 105°C pendant 72 heures et transformation des acides aminés libérés en dérivés phenylthiohydantoïne (PTH-aa). Le nombre moyen d'ANP par molécule de polymère est 8.

**Préparation de la polylysine histidylée substituée par la biotine**

La polylysine substituée par 60 résidus histidyles (15 mg; 0,28 μmol) dissoute dans 1 ml de DMSO en pésence de DIEA (4 μl; 28 μmol) est mise à réagir pendant 7 h à 20°C avec l'ester N-hydroxysuccinimide du 6-(biotinamido) hexanoate (NHS-LC-biotine, Pierce, USA). Le polymère est précipité par addition de 10 volumes d'isopropanol. Le précipité est récupéré après centrifugation (1 800 g, 15 min) et lavé dans l'isopropanol puis dissous dans l'eau et lyophilisé.

## Revendications

1. Complexe entre au moins un acide nucléique (chargé négativement) et au moins un conjugué polymérique chargé positivement, la liaison entre l'acide nucléique et le conjugué polymérique étant de nature électrostatique, le conjugué polymérique contenant un polymère formé de motifs monomères portant des fonctions $NH_3^+$ libres, et étant tel que :

   - les fonctions $NH_3^+$ libres des susdits motifs monomères sont substituées dans un rapport d'au moins 10%, par des résidus protonables dans un milieu dont le pH est inférieur à celui du plasma ou du sérum, lesdits résidus entraînant dans un milieu dont le pH est inférieur à celui du plasma ou du sérum une déstabilisation des membranes cellulaires,
   - les susdits résidus possédant en outre les propriétés suivantes :

     . ils comportent un groupe fonctionnel leur permettant d'être fixés au susdit polymère,
     . ils ne sont pas actifs en tant que signal de reconnaissance reconnu par un récepteur membranaire cellulaire,
     . ils appartiennent à la famille des composés comportant un noyau imidazole,
     . ils appartiennent à la famille des quinolines,
     . ils appartiennent à la famille des ptérines,
     . ils appartiennent à la famille des pyridines.

   sous réserve que l'ensemble des fonctions $NH_3^+$ libres, soit d'au moins 30% du nombre des motifs monomères du squelette polymérique du susdit conjugué polymérique,

   et sous réserve que le conjugué polymérique soit différent du conjugué polylysine - (dithiopyridyl)propionate.

2. Complexe selon la revendication 1, dans lequel les résidus protonables dans un milieu dont le pH est inférieur à celui du plasma ou du sérum comportent au moins une fonction $NH_3^+$ libre.

3. Complexe selon la revendication 1 ou 2, dans lequel des molécules constituant un signal de reconnaissance reconnu par un récepteur membranaire cellulaire sont présentes :

   . soit par substitution de certaines des fonctions $NH_3^+$ libres des motifs monomères,
   . soit sur certains des résidus entraînant une déstabilisation des membranes cellulaires,
   . soit par substitution de la fonction $NH_3^+$ libre des résidus entraînant une déstabilisation des membranes cellulaires.

4. Complexe selon l'une des revendications 1 à 3, **caractérisé en ce que** les fonctions $NH_3^+$ libres des motifs monomères sont substituées dans un rapport d'environ 15% à environ 45%.

5. Complexe selon l'une des revendications 1 à 4, dans lequel les résidus protonables dans un milieu dont le pH est inférieur à celui du plasma ou du sérum sont des bases dont le pK en milieu aqueux est inférieur à 8, de sorte qu'une proportion supérieure à 50% de ces bases liée à un polymère cationique ne soit pas protonée en milieu neutre de pH 7,4.

6. Complexe selon l'une quelconque des revendications 1 à 5, dans lequel les résidus, protonables dans un milieu dont le pH est inférieur à celui du plasma ou du sérum, entraînant une déstabilisation des membranes cellulaires sont choisis parmi :

   histidine, 4-carboxyméthyl-imidazole, 3-(1-méthyl-imidazol-4yl)-alanine, 3-(3-méthyl-imidazol-4yl)-alanine, 2-carboxy-imidazole, histamine, acide 3-(imidazol-4yl)-L-lactique, 2-(1-méthyl-imidazol-4yl)éthylamine, 2-(3-méthyl-imidazol-4yl)éthylamine, β-alanyl-histidine-(carnosine), 7-chloro-4(amino-1-methylbutylamino)-quinoline, $N^4$-(7-chloro-4-quinolinyl)-1,4-pentanediamine, 8-(4-amino-1-méthylbutylamino)-6-méthoxy-quinoline (primaquine), $N^4$-(6-méthoxy-8-quinolinyl)1,4-pentanediamine, acide quininique, acide quinoline carboxylique, acide ptéroïque, acide nicotinique, acide quinolinique.

7. Complexe selon l'une des revendications 1 à 6, **caractérisé en ce que** les fonctions $NH_3^+$ portées par les motifs monomères sont des résidus de lysine ou d'omithine, et **en ce que** :

- les fonctions $NH_3^+$ libres restantes des susdits motifs monomères sont également substituées à raison d'environ 1% à environ 60% par une molécule qui constitue un signal de reconnaissance reconnu par un récepteur membranaire cellulaire, ce signal de reconnaissance ayant une masse moléculaire inférieure à 5000,

  sous réserve que l'ensemble des fonctions $NH_3^+$ libres soit d'au moins 30% du nombre des motifs monomères du squelette polymérique du susdit conjugué polymérique.

**8.** Complexe selon l'une des revendications 1 à 5, dans lequel le polymère contient un groupement polymérique de formule (I) suivante :

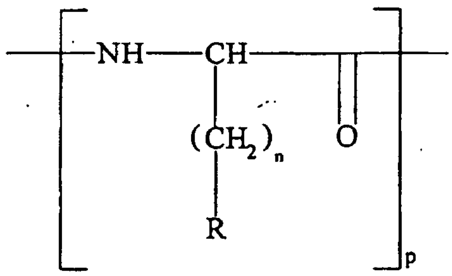

(I)

dans laquelle :

- p est un nombre entier variant de 15 à 900,
- n est un nombre entier variant de 1 à 6,
- ce groupement polymérique contient des radicaux R parmi lesquels :
  . 10% à 45% du nombre de radicaux R représentent un résidu comportant un noyau imidazole
  . 10% à 90% du nombre de radicaux R, représentent les $\overline{\omega}$-amino $NH_3^+$ libres.

**9.** Complexe selon la revendication 8, **caractérisé en ce que** R est représenté par la formule :

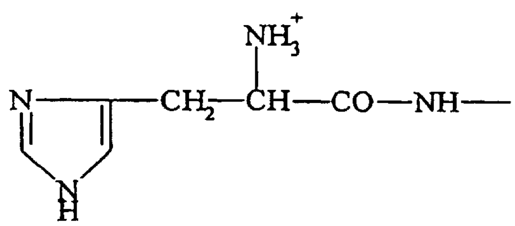

**10.** Complexe selon la revendication 8 ou 9, dans lequel 10% à 90% du nombre de radicaux R, représentant les $\overline{\omega}$-amino $NH_3^+$ libres, est substitué à raison de 0 à 50% par une molécule qui constitue un signal de reconnaissance.

**11.** Complexe selon la revendication 8 à 10, dans lequel le polymère comprend un groupement polymérique de formule (II) suivante :

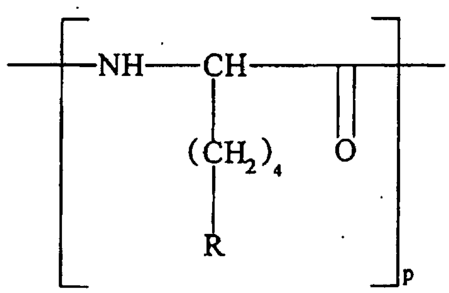

dans laquelle :

- p a les significations indiquées dans la revendication 8,
- 10% à 45% du nombre de radicaux R représentent un résidu comportant un noyau imidazole.

**12.** Complexe selon la revendication 11, **caractérisé en ce que** le reste des radicaux R, c'est-à-dire 30% à 90% du nombre de radicaux R, représentent les $\bar{\omega}$-amino $NH_3^+$.

**13.** Complexe selon la revendication 12, **caractérisé en ce que** de 0% à 45% des radicaux R sont substitués par une molécule qui constitue un signal de reconnaissance reconnu par un récepteur membranaire cellulaire.

**14.** Complexe selon l'une des revendications 1 à 6, **caractérisé en ce que** le signal de reconnaissance est choisi parmi :

A) des osides simples ou complexes reconnus par des lectines membranaires, et choisis parmi :

a. Asialo-oligoside de type triantenné lactosamine : récepteur d'asialoglycoprotéine

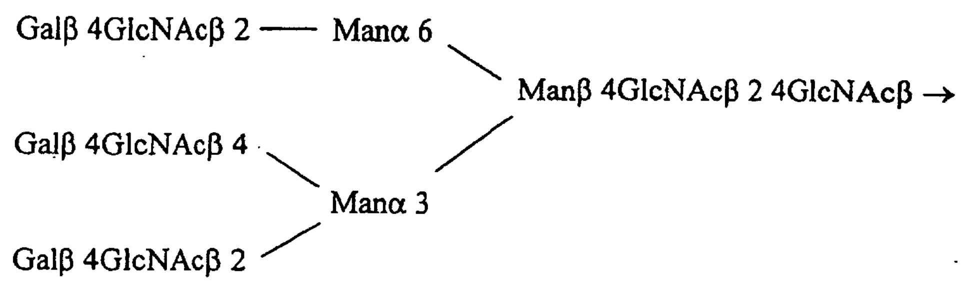

b. Asialo oligoside de type lactosamine tetraantenné : récepteur d'asialoglycoprotéine

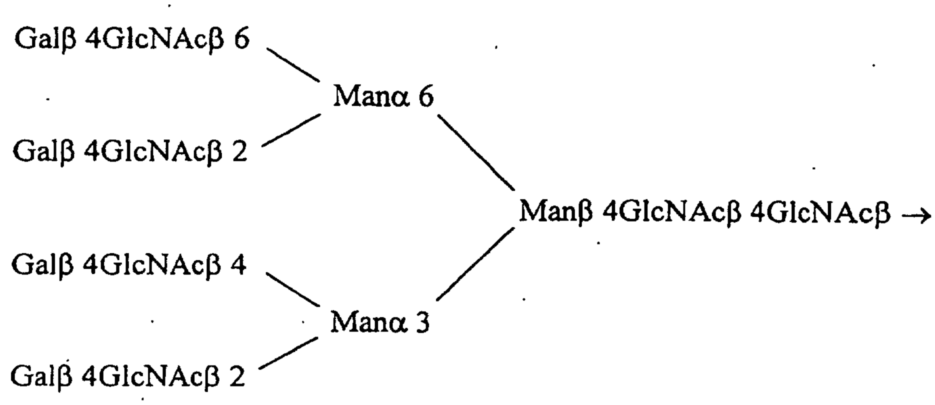

c. Lewis x :

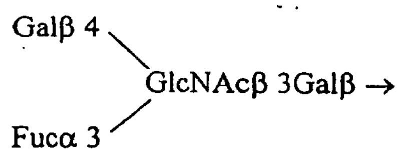

d. Lewis x sialyl : LECAM 3/2

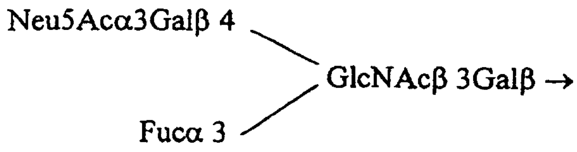

e. Dérivé de Lewis x sulfaté (HNK1) :

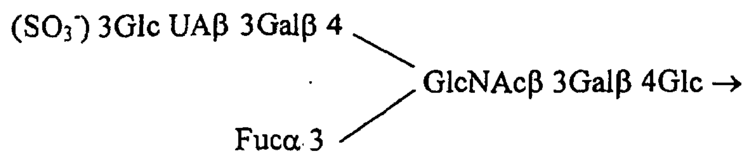

f. Oligomannoside : récepteur du mannose

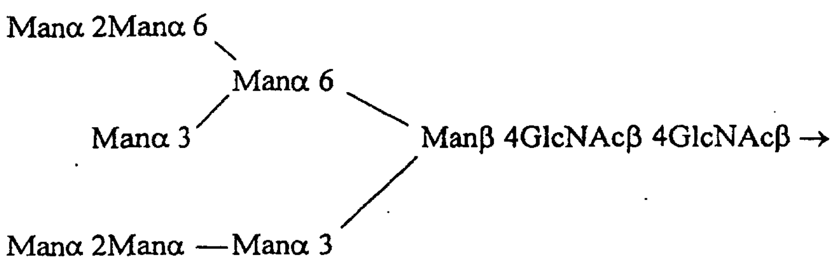

g. Oligomannoside phosphorylé : récepteur de mannose 6 phosphate

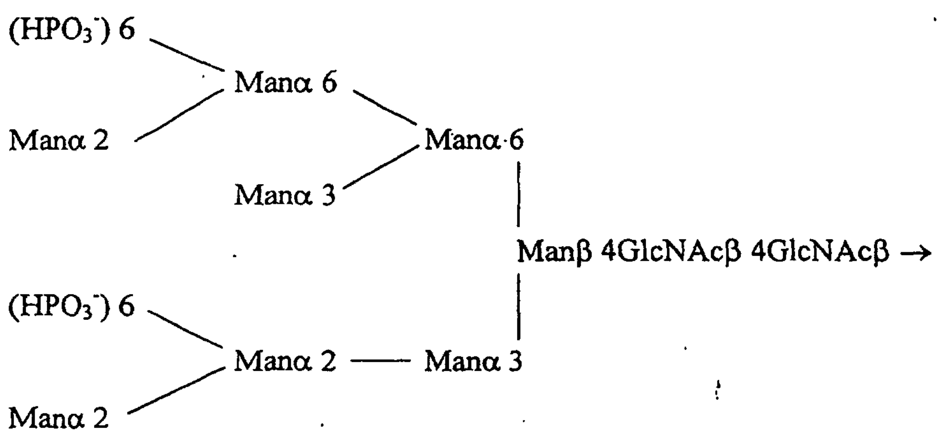

h. Oligosaccharide de type lactosamine sulfatée : récepteur de GalNAc 4 sulfaté

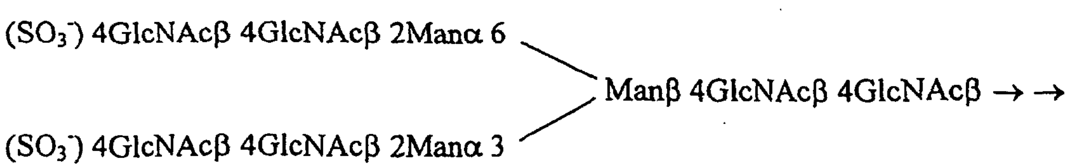

B) des peptides

a) peptides anti-inflammatoires ou certains de leurs fragments reconnus par des récepteurs de la paroi vasculaire,
b) peptides ligands des intégrines,
c) facteurs chimiotactiques,
d) hormones peptidiques

C) Métabolites naturels.

**15.** Complexe selon l'une des revendications 1 à 7, **caractérisé en ce que** l'acide nucléique est choisi parmi :

a) des gènes marqueurs,
b) des gènes à visée thérapeutique,
c) des gènes à visée vaccinale

**16.** Complexe selon la revendication 15, **caractérisé en ce que** les gènes marqueurs sont choisis parmi : les gènes contenant la luciférase, la protéine verte de la méduse *Aequorea victoria*, les gènes contenant la β-galactosidase, les gènes contenant la chloramphénicol acétyl transférase et les gènes conférant la résistance à un antibiotique.

**17.** Complexe selon la revendication 15, **caractérisé en ce que** les gènes à visée thérapeutique sont choisis parmi :

- récepteurs des lipoprotéines de faible densité, déficient dans les cas d'hypercholestérolémie,
- facteurs de coagulation : facteurs VIII et IX,
- phénylalanine-hydroxylase (phénylcétonurie),
- adénosine désaminase (immunodéficience ADA),
- enzymes lysosomiques, telles que la β-glucosidase dans le cas de la maladie de Gaucher,
- dystrophine et minidistrophine (myopathie),
- tyrosine hydroxylase (Parkinson),
- facteurs de croissance des neurones (Alzheimer),
- CFTR cystic-fibrosis transmembrane conductance regulator (mucoviscidose),
- alphal-antitrypsine,
- cytokines (interleukines, TNF facteur de nécrose des tumeurs),
- thymidine kinase du virus Herpes simplex,
- protéines du MHC, système majeur d'histocompatibilité, en particulier les HLA-B7,
- cytosine désaminase,
- gènes codant pour des ARN sens et antisens,
- gènes codant pour des ribozymes.

**18.** Complexe selon la revendication 15, **caractérisé en ce que** les gènes à visée vaccinale sont choisis parmi les gènes codant pour des antigènes viraux (vaccination).

**19.** Complexe selon l'une des revendications 1 à 10, dans lequel :

- le polymère est la polylysine et présente un degré de polymérisation d'environ 15 à environ 900,
- les fonctions $NH_3^+$ libres des motifs lysine étant substituées dans un rapport de 35% par des résidus histidyle,
- l'acide nucléique présente une masse moléculaire d'environ $10^6$ à environ $10^8$,
- le rapport entre le nombre moyen de paires de base de l'acide nucléique par molécule de motif de lysine est d'environ 0,2 à environ 6.

**20.** Utilisation d'un complexe. selon l'une des revendications 1 à 19, pour la préparation d'un médicament destiné à la transfection *in vitro, ex vivo* ou *in vivo* de cellules à l'aide d'un gène, notamment ceux définis à la revendication 17 ou 18.

**21.** Utilisation d'un complexe selon la revendication 19, **caractérisé en ce que** les cellules sont choisies parmi :

- des cellules souches hématopoïétiques ;
- des cellules dendritiques ;

- cellules du foie ;
- cellules des muscles squelettiques ;
- cellules de la peau :
    . fibroblastes,
    . kératinocytes,
    . cellules dendritiques,
    . mélanocytes,
- cellules des parois vasculaires ;
    . endothéliales ;
    . musculaires lisses ;
- cellules épithéliales des voies aériennes ;
- cellules du système nerveux central ;
- cellules cancéreuses ;
- cellules du système immunitaire.

**22.** Méthode de transfection *in vitro* ou *ex vivo*, **caractérisée en ce que** l'on met en présence un complexe, selon l'une quelconque des revendications 1 à 19, dans un milieu contenant des cellules à transfecter, dans des conditions telles qu'il y a :

- passage du complexe à partir du milieu dans le cytoplasme des cellules,
- relargage de l'acide nucléique impliqué dans le susdit complexe dans le cytosol et/ou le noyau des cellules,
- transcription et expression de l'acide nucléique dans les cellules transfectées
- expression de la protéine correspondant au gène transfecté.

**23.** Composition pharmaceutique, **caractérisée en ce qu'**elle comprend à titre de substance active, l'un au moins des complexes selon l'une quelconque des revendications 1 à 19, en association avec un véhicule pharmaceutiquement acceptable.

**24.** Utilisation d'un complexe selon l'une des revendications 1 à 19, pour la préparation d'un médicament destiné par exemple au traitement de déficience métabolique congénitale ou acquise, ou au traitement de tumeurs, ou pour la préparation d'un vaccin, par exemple vaccin contre la grippe.

**Patentansprüche**

**1.** Komplex zwischen mindestens einer Nukleinsäure (negative Ladung) und mindestens einem positiv geladenen Polymerkonjugat, wobei die Bindung zwischen der Nukleinsäure und dem Polymerkonjugat elektrostatischer Natur ist, das Polymerkonjugat ein Polymer ist, das durch Monomere gebildet ist, die freie $NH_3^+$-Funktionen tragen und so ist, dass:

- die freien $NH_3^+$-Funktionen der vorher erwähnten Monomere in einem Anteil von mindestens 10% durch Reste substituiert sind, die in einem Medium, dessen pH geringer als der des Plasmas oder des Serums ist, protonierbar sind, wobei die erwähnten Reste in einem Medium, dessen pH geringer als der des Plasmas oder des Serums ist, eine Destabilisierung der Zellmembranen verursachen,
- wobei die vorher erwähnten Reste unter anderem die folgenden Eigenschaften besitzen:
    . sie enthalten eine funktionelle Gruppe, die es ihnen erlaubt, an die vorher erwähnten Polymere fixiert zu sein,
    . sie sind nicht als ein durch einen zellulären Membranrezeptor erkanntes Erkennungssignal aktiv,
    . sie gehören zu der Familie von Verbindungen, welche einen Imidazolkern enthalten,
    . sie gehören zu der Familie der Chinoline,
    . sie gehören zu der Familie der Pterine,
    . sie gehören zu der Familie der Pyridine,

unter der Voraussetzung, dass die freien $NH_3^+$-Funktionen insgesamt mindestens 30% der Anzahl der Monomere des Polymerskeletts des vorher erwähnten Polymerkonjugats sind, und unter der Voraussetzung, dass das Polymerkonjugat unterschiedlich zu dem Polylysin-(dithiopyridyl)propionat-Konjugat ist.

**2.** Komplex nach Anspruch 1, in dem die Reste, die in einem Medium protonierbar sind, dessen pH niedriger als der des Plasmas oder des Serums ist, mindestens eine freie $NH_3^+$-Funktion enthalten.

**3.** Komplex nach Anspruch 1 oder 2, in dem Moleküle vorhanden sind, die ein durch einen zellulären Membranrezeptor erkanntes Erkennungssignal bilden,
entweder durch Substitution bestimmter freier $NH_3^+$-Funktionen der Monomere,
oder durch bestimmte Reste, die eine Destabilisierung der Zellmembranen verursachen,
oder durch Substitution der freien $NH_3^+$-Funktionen der Reste, die eine Destabilisierung der Zellmembranen verursachen.

**4.** Komplex nach einem der Ansprüche 1 bis 3, **dadurch gekennzeichnet, dass** die freien $NH_3^+$-Funktionen der Monomere in einem Anteil von etwa 15% bis etwa 45% substituiert sind.

**5.** Komplex nach einem der Ansprüche 1 bis 4, wobei darin die Reste, die in einem Medium protonierbar sind, dessen pH niedriger als der des Plasmas oder des Serums ist, Basen sind, deren pK in einem wässrigen Medium niedriger als 8 ist, so dass ein größerer Anteil als 50% dieser Basen, der an ein kationisches Polymer gebunden ist, in einem neutralen Medium von pH 7,4 nicht protoniert wird.

**6.** Komplex nach einem der Ansprüche 1 bis 5, in welchem die in einem Medium protonierbaren Reste, dessen pH niedriger als der des Plasmas oder des Serums ist, die eine Destabilisierung der zellulären Membranen verursachen, ausgewählt sind aus:

Histidin, 4-Carboxymethyl-imidazol, 3-(1-Methyl-imidazol-4yl)-alanin, 3-(3-Methyl-imidazol-4yl)-alanin, 2-Carboxy-imidazol, Histamin, 3-(Imidazol-4yl)-L-Milchsäure, 2-(1-Methyl-imidazol-4yl)ethylamin, 2-(3-Methyl-imidazol-4yl)ethylamin, β-Alanyl-histidin-(carnosin), 7-Chlor-4(amino-1-methylbutylamin)-chinolin, $N^4$-(7-Chlor-4-chinolinyl)-1,4-pentandiamin, 8-(4-Amino-1-methylbutylamin)-6-methoxychinolin (Primachin), $N^4$-(6-Methoxy-8-chinolinyl)1,4-pentandiamin, Chininsäure, Chinolincarbonsäure, Pteroinsäure, Nikotinsäure, Chinolinsäure.

**7.** Komplex nach einem der Ansprüche 1 bis 6, **dadurch gekennzeichnet, dass** die durch die Monomere getragenen $NH_3^+$-Funktionen Lysin-Reste oder Omithin-Reste sind, und dass in ihnen:

- die verbleibenden freien $NH_3^+$-Funktionen der vorher erwähnten Monomere gleichmäßig entsprechend zu etwa 1% bis etwa 60% auf ein Molekül substituiert sind, welches ein durch einen zellulären Membranrezeptor erkanntes Erkennungssignal bildet, wobei das Erkennungssignal eine Molekülmasse unter 5000 hat,

unter der Voraussetzung, dass die Gesamtheit der freien $NH_3^+$-Funktionen wenigstens 30% der Anzahl der Monomere des Polymerskeletts des vorher erwähnten Polymerkonjugats ist.

**8.** Komplex nach einem der Ansprüche 1 bis 5, in dem das Polymer eine polymere Gruppe der folgenden Formel (I) enthält:

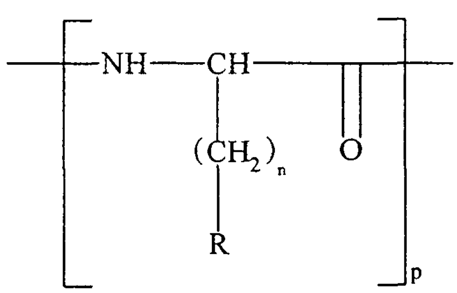

(I)

in der:

- p eine ganze Zahl von 15 bis 900 ist,
- n eine ganze Zahl ist von 1 bis 6 ist,
- die polymere Gruppe Radikale R enthält, unter denen:

  . 10% bis 45% der Anzahl der Radikale R einen Rest darstellen, der einen Imidazolkem umfasst,
  . 10% bis 90% der Anzahl der Radikale R die freien ω-Amino $NH_3^+$ darstellen.

**9.** Komplex nach Anspruch 8, **dadurch gekennzeichnet, dass** R durch die Formel dargestellt wird:

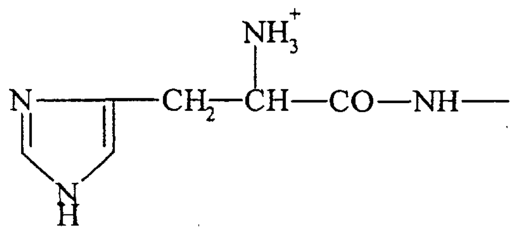

**10.** Komplex nach einem der Ansprüche 8 oder 9, in dem 10% bis 90% der Anzahl der Radikale R die freien ω-Amino $NH_3^+$ darstellen, wobei sie zu 0 bis 50% durch ein Molekül substituiert sind, welches ein Erkennungssignal bildet.

**11.** Komplex nach Anspruch 8 bis 10, in dem das Polymer eine polymere Gruppe der folgenden Formel (II) umfasst:

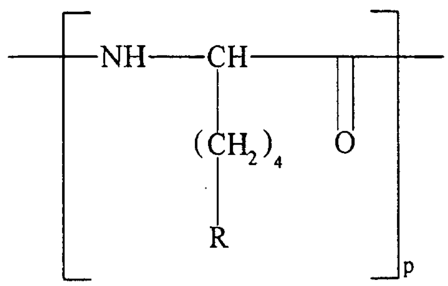

in der:

- p die im Anspruch 8 angegebene Bedeutung hat,
- 10% bis 45% der Anzahl der Radikale R einen Rest darstellen, der einen Imidazolkem enthält.

**12.** Komplex nach Anspruch 11, **dadurch gekennzeichnet, dass** die verbleibenden Radikale R, das heißt 30% bis 90% der Anzahl der Radikale R, ω-Amino $NH_3^+$ darstellen.

**13.** Komplex nach Anspruch 12, **dadurch gekennzeichnet, dass** 0% bis 45% der Radikale R durch ein Molekül substituiert sind, welches ein durch einen zellulären Membranrezeptor erkanntes Erkennungssignal bildet.

**14.** Komplex nach einem der Ansprüche 1 bis 6, **dadurch gekennzeichnet, dass** das Erkennungssignal ausgewählt ist aus:

A) einfache oder komplexe Oside, welche durch die Membranlektine erkannt werden und ausgewählt werden aus:

a. Asialooligosid des Triantennenlactosamin-Typs: Asialoglykoprotein-Rezeptor,

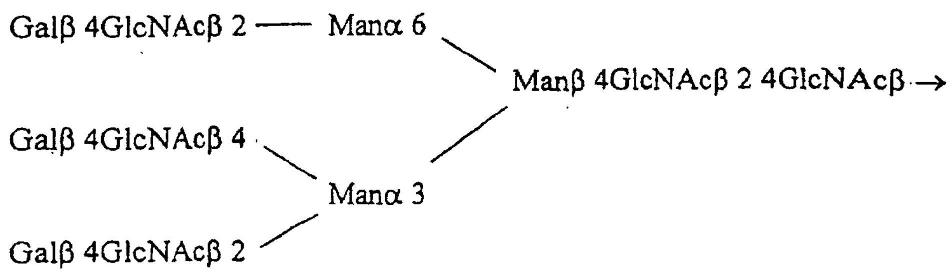

b. Asialooligosid des Tetraantennenlactosamin-Typs: Asialoglykoprotein-Rezeptor

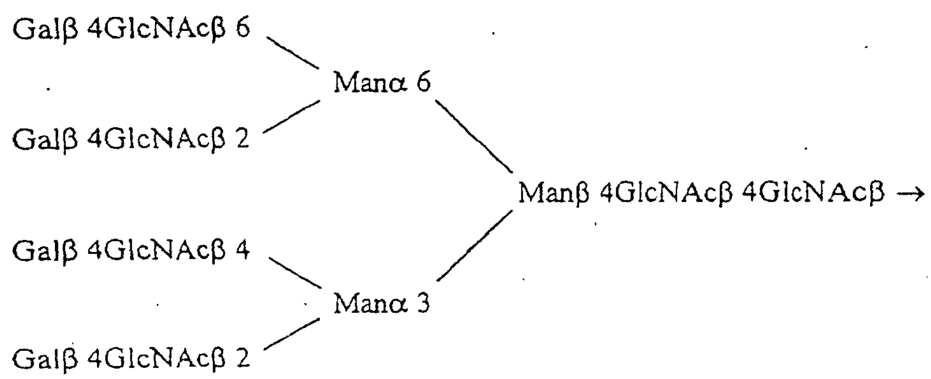

c. Lewis x:

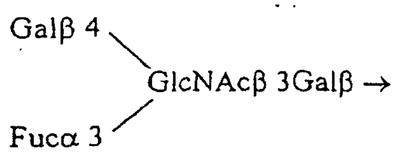

d. Lewis x Sialyl: LECAM 3/2

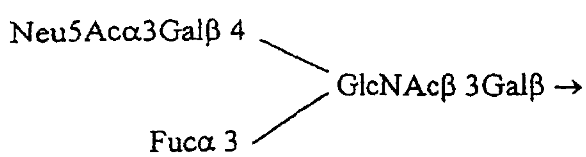

e. Derivate des Lewis-x-Sulfats (HNK1):

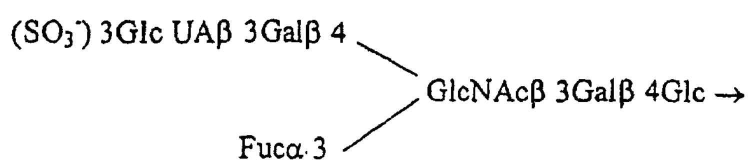

f. Oligomannosid: Mannose-Rezeptor

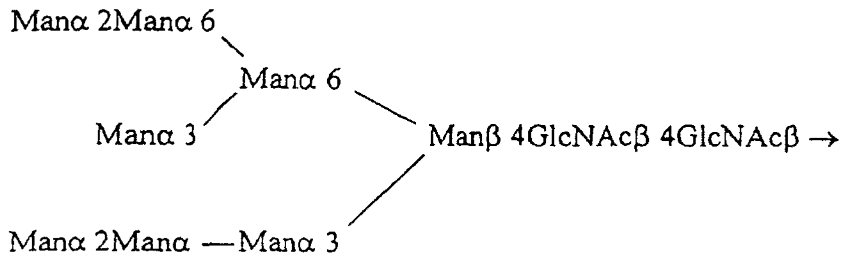

g. Phosphoryliertes Oligomannosid: Mannose-6-Phosphat-Rezeptor

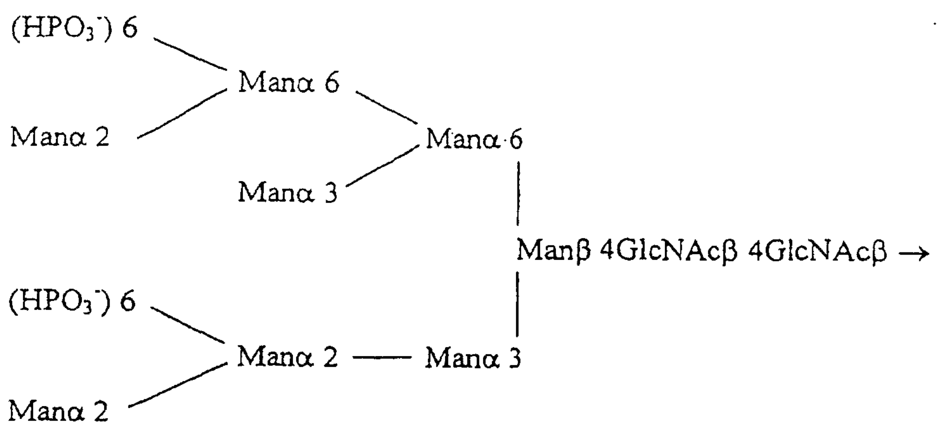

h. Oligosaccharid vom sulfatierten Lactosamin-Typ:

Rezeptor des sulfatierten GalNAc-4

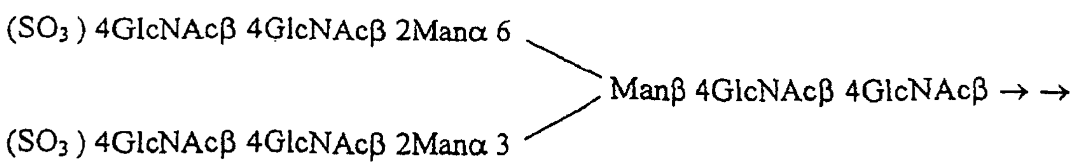

B) Peptide

a) Antiinflammatorische Peptide oder bestimmte ihrer Fragmente, die durch die Rezeptoren der Gefäßwand erkannt werden,
b) Ligandpeptide der Integrine,
c) Chemotaktische Faktoren,
d) Peptidhormone

C) natürliche Metabolite.

**15.** Komplex nach einem der Ansprüche 1 bis 7, **dadurch gekennzeichnet, dass** die Nukleinsäure ausgewählt ist aus:

a) Markergenen,
b) therapeutische Zielgene,
c) Vakzine-Zielgene.

**16.** Komplex nach Anspruch 15, **dadurch gekennzeichnet, dass** die Markergene ausgewählt sind aus: den Luciferase-Genen, denen das grüne Protein der Meduse *Aequorea victoria* enthaltenden Genen, den β-Galactosidase enthaltenden Genen, den Chloramphenicolacetyltransferase enthaltenden Genen und den eine Antibiotika-Resistenz vermittelnden Genen.

**17.** Komplex nach Anspruch 15, **dadurch gekennzeichnet, dass** die therapeutischen Zielgene ausgewählt sind aus:

- Rezeptoren von Lipoproteinen geringer Dichte, die im Fall der Hypercholesterolemie defekt sind,
- Gerinnungsfaktoren: Faktoren VIII und IX,
- Phenylalanin-Hydroxylase (Phenylketonurie),
- Adenosindesaminase (Immundefizienz ADA),
- Lysosomenzyme, wie die β-Glucosidase im Fall der Gaucher-Krankheit,
- Dystrophin und Minidistrophin (Myopathie),
- Tyrosinhydroxylase (Parkinson),
- Kreuzfaktoren der Neuronen (Alzheimer),
- CFTR zystisch fibrotischer Transmembranleitfähigkeitsregulator (Mukoviszidose),
- Alphal-Antitrypsin,
- Zytokine (Interleukine, TNF Tumomekrosefaktor),
- Thymidinkinase des Herpes-simplex-Virus,
- Proteine des MHC, Haupthistokompatibilitätsystems, und insbesondere die HLA-B7,
- Zytosindesaminase,
- Gene, die für Sense- und Antisense-RNA kodieren,
- Gene, die für Ribozyme kodieren.

**18.** Komplex nach Anspruch 15, **dadurch gekennzeichnet, dass** die Vakzine-Zielgene ausgewählt sind aus den Genen, die für virale Antigene kodieren (Vakzination).

**19.** Komplex nach einem der Ansprüche 1 bis 10, in dem:

- das Polymer Polylysin ist und ein Polymerisationsgrad von etwa 15 bis etwa 900 aufweist,
- die freien $NH_3^+$-Funktionen der Lysine in einem Anteil von 35% durch Histidyl-Reste substituiert sind,
- die Nukleinsäure eine Molekülmasse von etwa $10^6$ bis $10^8$ aufweist,
- die Beziehung zwischen der mittleren Anzahl der Basenpaare der Nukleinsäure pro Lysinmolekül etwa zwischen 0,6 bis etwa 6 ist.

**20.** Verwendung eines Komplexes nach einem der Ansprüche 1 bis 19 für die Herstellung eines Medikaments zur Transfektion *in vitro*, *ex vivo* oder *in vivo* von Zellen mit der Hilfe eines Genes, insbesondere den in den Ansprüchen 17 und 18 definierten.

**21.** Verwendung eines Komplexes nach Anspruch 19, **dadurch gekennzeichnet, dass** die Zellen ausgewählt sind aus:

- hämatopoetischen Stammzellen,
- dendritischen Zellen,
- Leberzellen,
- Skelettmuskelzellen,
- Hautzellen:
  - . Fibroblasten,
  - . Keratinozyten,
  - . dendritischen Zellen,
  - . Melanozyten,
- Gefäßwandzellen,
  - . endothel,
  - . glatte Muskulatur,
- Epithelzellen der Atmungsorgane

- Zellen des zentralen Nervensystems,
- Krebszellen,
- Zellen des Immunsystems.

**22.** Verfahren für die Transfektion *in vitro* oder *ex vivo*, **dadurch gekennzeichnet, dass** es in der Anwesenheit eines Komplexes nach einem der Ansprüche 1 bis 19 durchgeführt wird, in einem Medium, welches zu transfizierende Zellen unter Bedingungen enthält, die wie folgt sind:

- Passage des Komplexes vom Medium in das Zytoplasma der Zellen,
- Befreien der in dem vorher erwähnten Komplex enthaltenden Nukleinsäure im Cytosol und/oder im Kern der Zellen,
- Transkription und Expression der Nukleinsäure in den transfizierten Zellen,
- Expression der zum transfizierten Gen korrespondierenden Protein.

**23.** Pharmazeutische Zusammensetzung, **dadurch gekennzeichnet, dass** es als Wirkstoff wenigstens einen der Komplexe nach einem der Ansprüche 1 bis 19 zusammen mit einem pharmazeutisch akzeptablen Träger enthält.

**24.** Verwendung eines Komplexes nach einem der Ansprüche 1 bis 19 für die Herstellung eines Medikaments, beabsichtigt z.B. für die Behandlung vererbter oder erworbener metabolischer Störungen oder zur Behandlung von Tumoren oder zur Herstellung einer Vakzine, z.B. eine Vakzine gegen die Grippe.

## Claims

**1.** Complex between at least one (negatively charged) nucleic acid and at least one positively charged polymeric conjugate, the bond between the nucleic acid and the polymeric conjugate being electrostatic in nature and the polymeric conjugate containing a polymer formed from monomer units carrying free $NH_3^+$ functions, and being such that:

- the free $NH_3^+$ functions of the abovementioned monomer units are substituted in a ratio of at least 10% by residues which can be protonated in a medium, the pH of which is less than that of plasma or serum, said residues causing destabilization of cell membranes in a medium, the pH of which is less than that of plasma or serum,
- the abovementioned residues also presenting the following properties:

  . they carry a functional group which enables them to be bound to the abovementioned polymer,
  . they are not active with respect to the recognition signal recognized by a cell membrane receptor,
  . they belong to the family of compounds which carry an imidazole nucleus,
  . they belong to the family of quinolines,
  . they belong to the family of pterines,
  . they belong to the family of pyridines,

with the proviso that the totality of the free $NH_3^+$ functions is of at least 30% of the number of monomer units of the polymeric skeleton of the abovementioned polymeric conjugate,

and with the proviso that the polymeric conjugate is different from the polylysine-(dithiopyridyl)propionate conjugate.

**2.** Complex according to claim 1, wherein the residues which can be protonated in a medium, the pH of which is less than that of plasma or serum carry at least one free $NH_3^+$ function.

**3.** Complex according to claim 1 or 2, wherein molecules constituting a recognition signal recognized by a cell membrane receptor are present:

. either by substitution of some of the free $NH_3^+$ functions of the abovementioned monomer units,
. or on some of the abovementioned residues causing a destabilization of cell membranes,
. or by substitution of the free $NH_3^+$ function of the residues causing a destabilization of cell membranes.

**4.** Complex according to one of claims 1 to 3, **characterized in that** the free $NH_3^+$ functions of the monomer units

are substituted in a ratio of about 15% to about 45%.

**5.** Complex according to one of claims 1 to 4, wherein the residues which can be protonated in a medium, the pH of which is less than that of plasma or serum are bases, the pK of which in an aqueous medium is less than 8, such that a proportion greater than 50% of these bases bound to a cationic polymer is not protonated in a neutral medium of pH 7.4.

**6.** Complex according to any one of claims 1 to 5, wherein the residues, which can be protonated in a medium, the pH of which is less than that of plasma or serum and causing destabilization of cell membranes are chosen from:

histidine, 4-carboxymethyl-imidazole, 3-(1-methyl-imidazol-4-yl)-alanine, 3-(3-methyl-imidazol-4-yl)-alanine, 2-carboxy-imidazole, histamine, 3-(imidazol-4-yl)-L-lactic acid, 2-(1-methyl-imidazol-4-yl)ethylamine, 2-(3-methyl-imidazol-4-yl) ethylamine, β-alanyl-histidine-(carnosine), 7-chloro-4-(amino-1 -methylbutylamino)-quinoline, $N^4$-(7-chloro-4-quinolinyl)-1,4-pentanediamine, 8-(4-amino-1-methylbutyl amino)-6-methoxyquinoline (primaquine), $N^4$-(6-methoxy-8-quinolinyl)-1,4-pentane diamine, quininic acid, quinolinecarboxylic acid, pteroic acid, nicotinic acid and quinolinic acid.

**7.** Complex according to any one of claims 1 to 6, **characterized in that** the $NH_3^+$ functions carried by monomer units are residues of lysine or omithine, and **in that**:

- the remaining free $NH_3^+$ functions of the abovementioned monomer units are also substituted to the extent of about 1% to about 60% by a molecule which constitutes a recognition signal recognized by a cell membrane receptor, this recognition signal having a molecular weight of less than 5,000,

with the proviso that the totality of the free $NH_3^+$ functions is of at least 30% of the number of monomer units of the polymeric skeleton of the abovementioned polymeric conjugate.

**8.** Complex according to any one of claims 1 to 5, in which the polymer contains a polymeric grouping of the following formula (I):

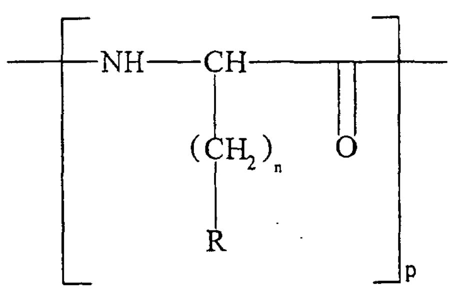

(I)

wherein:

- p is an integer varying from 15 to 900,
- n is an integer varying from 1 to 6,
- this polymeric grouping contains R moieties among which:
  - . 10% to 45% of the number of R moieties represent a residue carrying an imidazole nucleus
  - . 10% to 90% of the number of R moieties represent free $\bar{\omega}$-amino $NH_3^+$.

**9.** Complex according to claim 8, **characterized in that** R is represented by the formula:

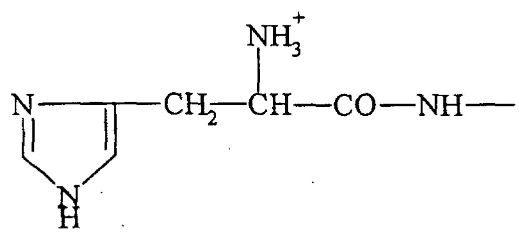

**10.** Complex according to claim 8 or 9, wherein 10% to 90% of the number of R moieties representing free $\bar{\omega}$-amino $NH_3^+$ are substituted to the extent of 0 to 50% by a molecule which constitutes a recognition signal.

**11.** Complex according to claim 8 to 10, in which the polymer comprises a polymeric grouping of the following formula (II):

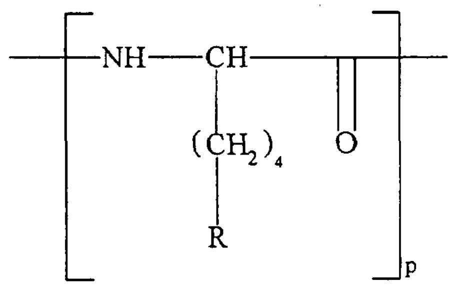

in which:

- p has the meanings indicated in claim 8,
- 10% to 45% of the number of R moieties represent a residue carrying an imidazole nucleus.

**12.** Complex according to claim 11, **characterized in that** the remainder of the radicals, that is to say 30% to 90% of the number of R moieties, represent $\bar{\omega}$-amino $NH_3^+$.

**13.** Complex according to claim 12, **characterized in that** 0 to 45% of the R moieties are substituted by a molecule which constitutes a recognition signal recognized by a cell membrane receptor.

**14.** Complex according to one of claims 1 to 6, **characterized in that** the recognition signal is chosen from:

A) simple or complex osides recognized by membrane lectins and chosen from:

a. Asialo-oligoside of the type of triantennar lactosamine: asialoglycoprotein receptor

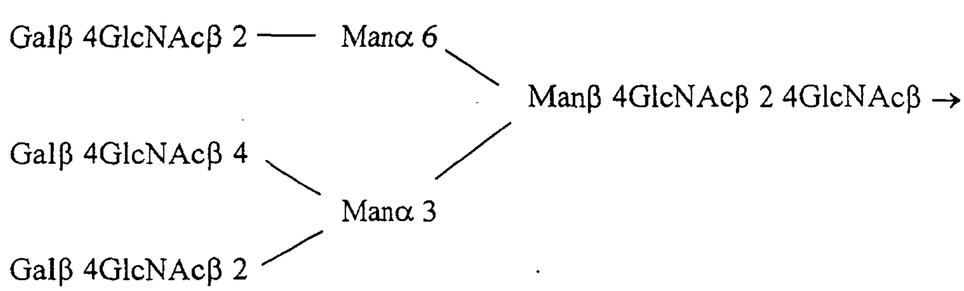

b. Asialo-oligoside of the type of tetraantennar lactosamine: asialoglycoprotein receptor

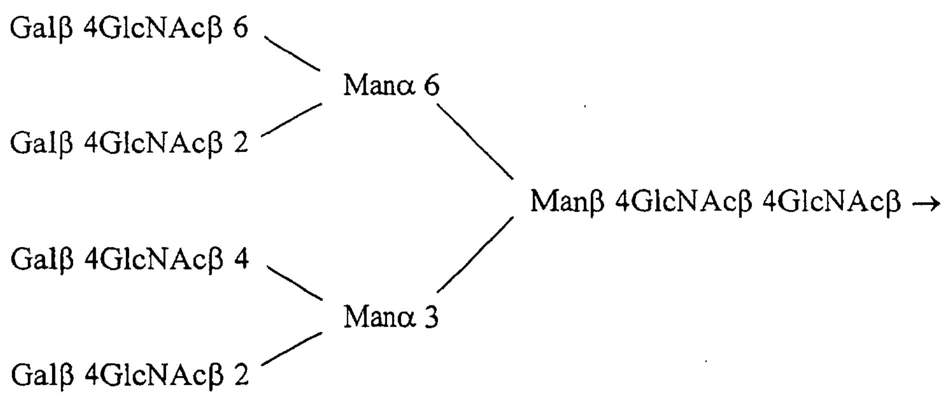

c. Lewis x:

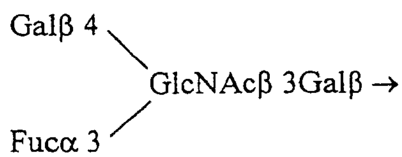

d. Lewis x sialyl: LECAM 3/2

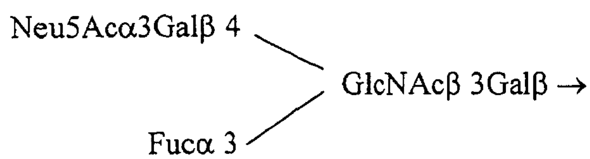

e. Sulphated Lewis x derivative (HNK1):

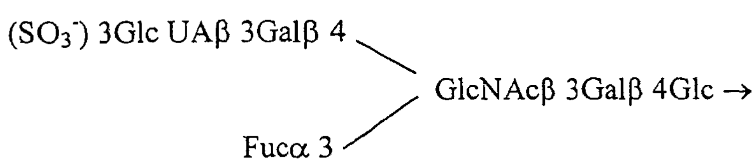

f. Oligomannoside: mannose receptor

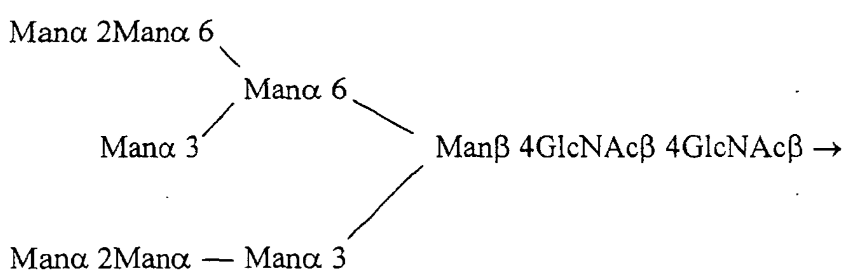

g. Phosphorylated oligomannoside: mannose 6-phosphate receptor

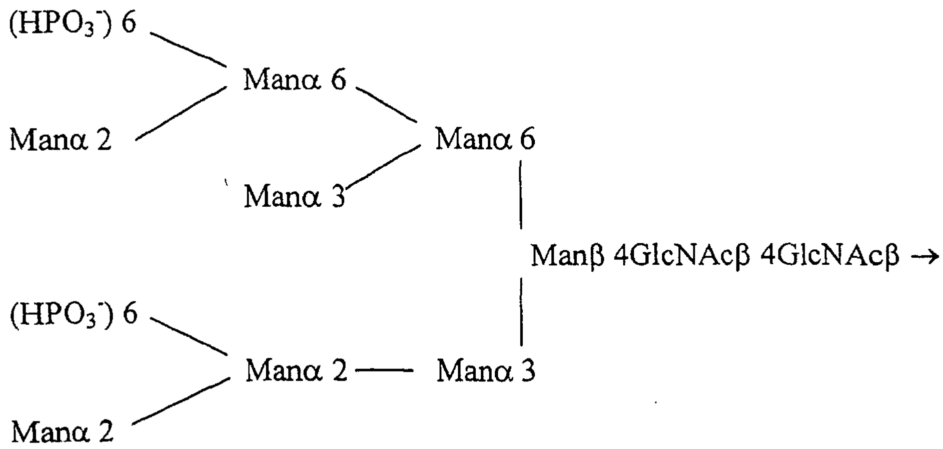

h. Oligosaccharide of the type of sulphated lactosamine: sulphated GalNAc 4 receptor

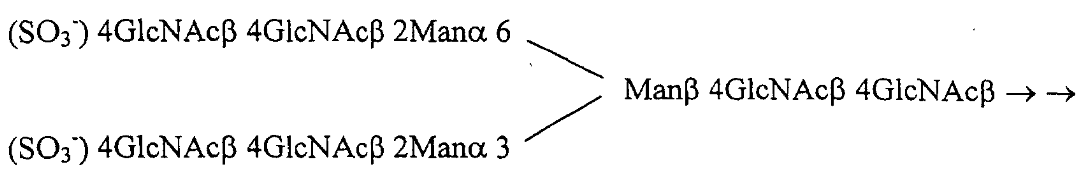

B) Peptides

a) anti-inflammatory peptides or certain of their fragments recognized by receptors of the vascular wall,
b) ligand peptides of integrins,
c) chemiotactic factors,
d) peptide hormones,

C) Natural metabolites.

**15.** Complex according to any one of claims 1 to 7, **characterized in that** the recognition signal is chosen from:

a) marker genes,
b) genes with a therapeutic purpose,
c) genes for the purpose of vaccines.

**16.** Complex according to claim 15, **characterized in that** marker genes are chosen from: genes containing luciferase, green protein of the jellyfish *Aequorea victoria*, genes containing β-galactosidase, genes containing chloramphenicol acetyltransferase, and genes which confer resistance to an antibiotic.

**17.** Complex according to claim 15, **characterized in that** genes with a therapeutic purpose are chosen from:

- receptors of low-density lipoproteins, which are deficient in cases of hypercholesterolaemia,
- coagulation factors: factors VIII and IX,
- phenylalanine hydroxylase (phenylketonuria),
- adenosine deaminase (ADA immunodeficiency),
- lysosomal enzymes, such as β-glucosidase in the case of Gaucher's disease,
- dystrophin and minidistriphin (myopathy),
- tyrosine hydroxylase (Parkinson),

- neurone growth factors (Alzheimer),
- CFTR cystic fibrosis transmembrane conductance regulator (cystic fibrosis),
- alpha-1-antitrypsin,
- cytokines (interleukins, TNF tumour necrosing factor),
- thymidine kinase of the Herpes simplex virus,
- proteins of MHC, major histocompatibility complex, in particular HLA-B7,
- cytosine deaminase,
- genes which code for sense and antisense RNAs,
- genes which code for ribozymes.

**18.** Complex according to claim 15, **characterized in that** genes for the purpose of vaccines are chosen from genes which code for viral antigens (vaccination).

**19.** Complex according to any one of claims 1 to 10, wherein:

- the polymer is polylysine, and has a degree of polymerization of about 15 to about 900,
- the free $NH_3^+$ functions of the lysine units being substituted in a ratio of 35% by histidyl residues, and,
- the nucleic acid has a molecular weight of about $10^6$ to about $10^8$,
- the ratio between the average number of base pairs of the nucleic acid per molecule of lysine unit is about 0.2 to about 6.

**20.** Use of a complex according to any one of claims 1 to 19 for the *in vitro, ex vivo* or *in vivo* transfection of cells with the aid of a gene, in particular those defined in claims 17 or 18.

**21.** Use of a complex according to claim 19, **characterized in that** the cells are chosen from:

- cells of haematopoietic strains;
- dendritic cells;
- liver cells;
- skeletal muscle cells;
- skin cells:
  - . fibroblasts,
  - . keratinocytes,
  - . dendritic cells,
  - . melanocytes;
- cells of the vascular walls;
  - . endothelial;
  - . smooth muscle;
- epithelial cells of the respiratory tract;
- cells of the central nervous system;
- cancerous cells;
- cells of the immune system.

**22.** Method of *in vitro* or *ex vivo* transfection, **characterized in that** a complex according to any one of claims 1 to 19 is brought into contact with a medium containing cells to be transfected under conditions such that there is:

- passage of the complex from the medium into the cytoplasm of the cells,
- salting out of the nucleic acid involved in the abovementioned complex in the cytosol and/or the nucleus of the cells,
- transcription and expression of the nucleic acid in the transfected cells,
- expression of the protein corresponding to the transfected gene.

**23.** Pharmaceutical composition, **characterized in that** it comprises, as the active substance, at least one of the complexes according to any one of claims 1 to 19, in combination with a pharmaceutically acceptable vehicle.

**24.** Use of a complex according to one of claims 1 to 19, for the preparation of a medicament intended, for example, for the treatment of congenital or acquired metabolic deficiency, or the treatment of tumours, or for the preparation of a vaccine, for example a vaccine against influenza.

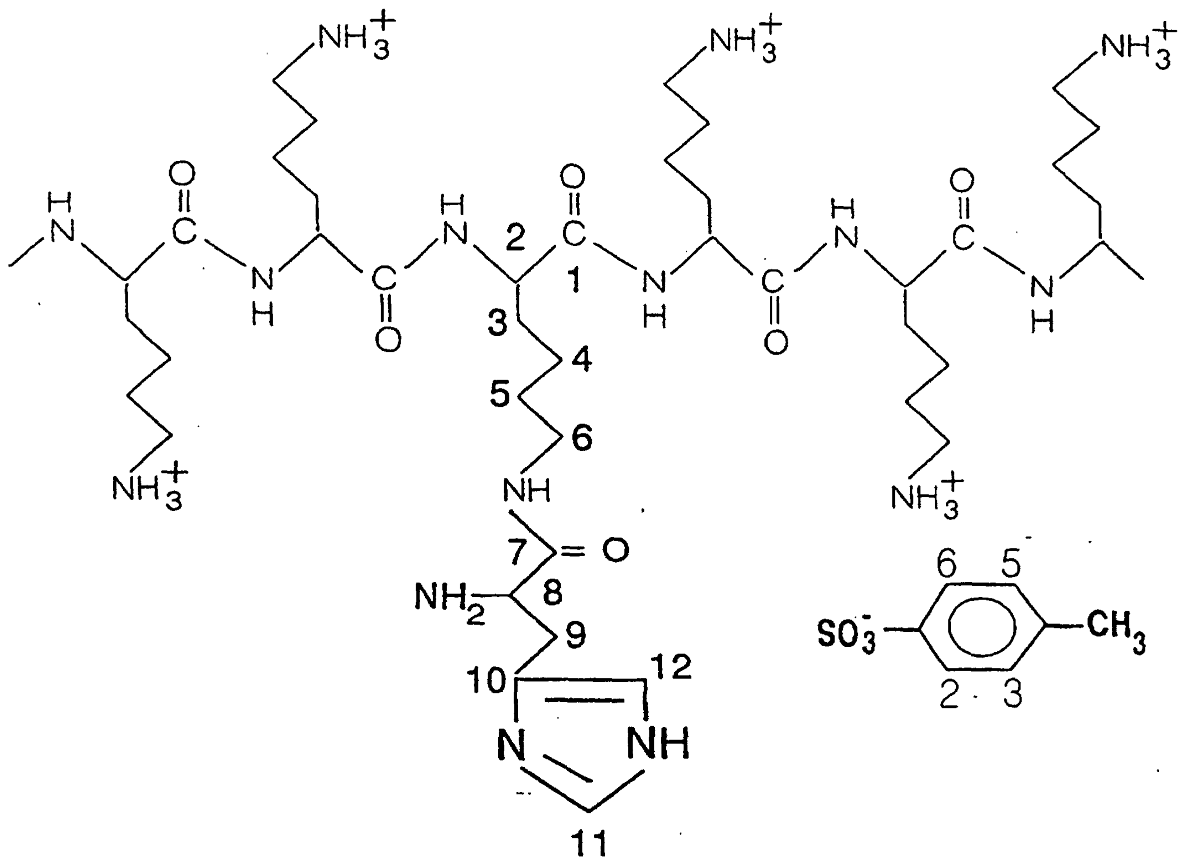

HispLK

Figure 1

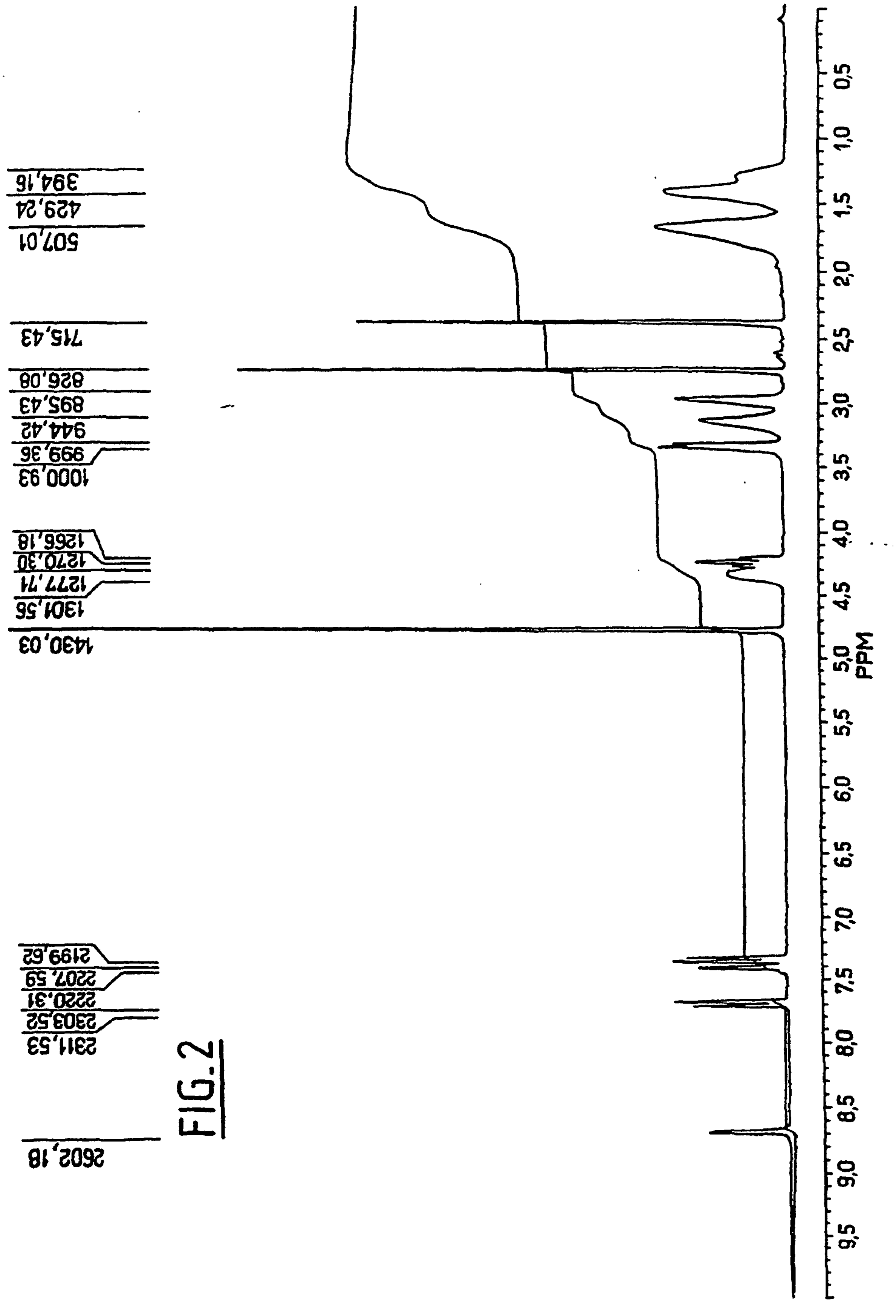
394,16
429,24
507,01
715,43
826,08
895,43
944,42
999,36
1000,93
1266,18
1270,30
1277,71
1301,56
1430,03
2199,62
2207,59
2220,31
2303,52
2311,53
2602,18
0,5
1,0
1,5
2,0
2,5
3,0
3,5
4,0
4,5
5,0
PPM
5,5
6,0
6,5
7,0
7,5
8,0
8,5
9,0
9,5

FIG.2

I

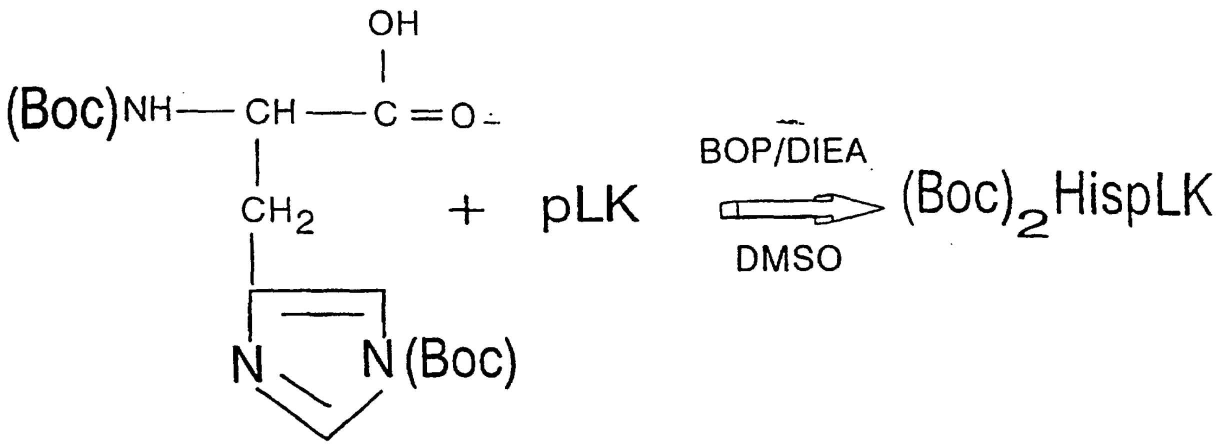


II

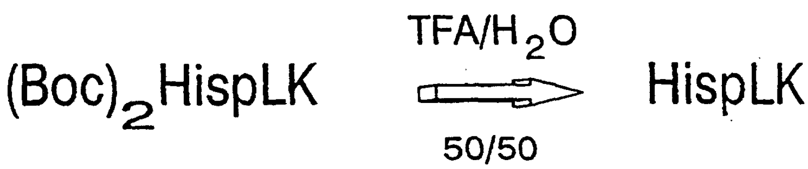


Figure 3

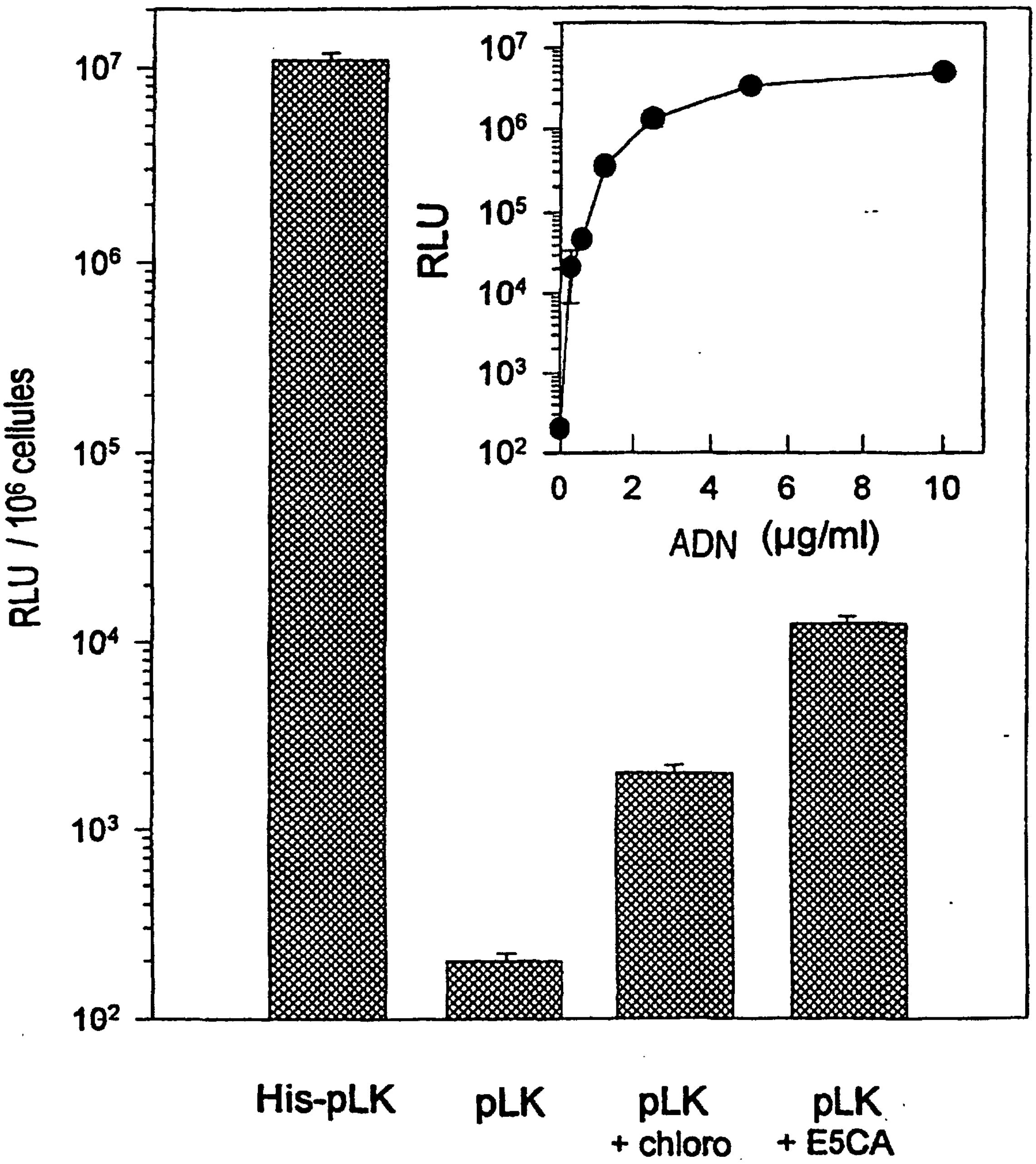
RLU / 10⁶ cellules
10²
10³
10⁴
10⁵
10⁶
10⁷
His-pLK
pLK
pLK + chloro
pLK + E5CA
RLU
ADN (µg/ml)
0
2
4
6
8
10

Figure 4

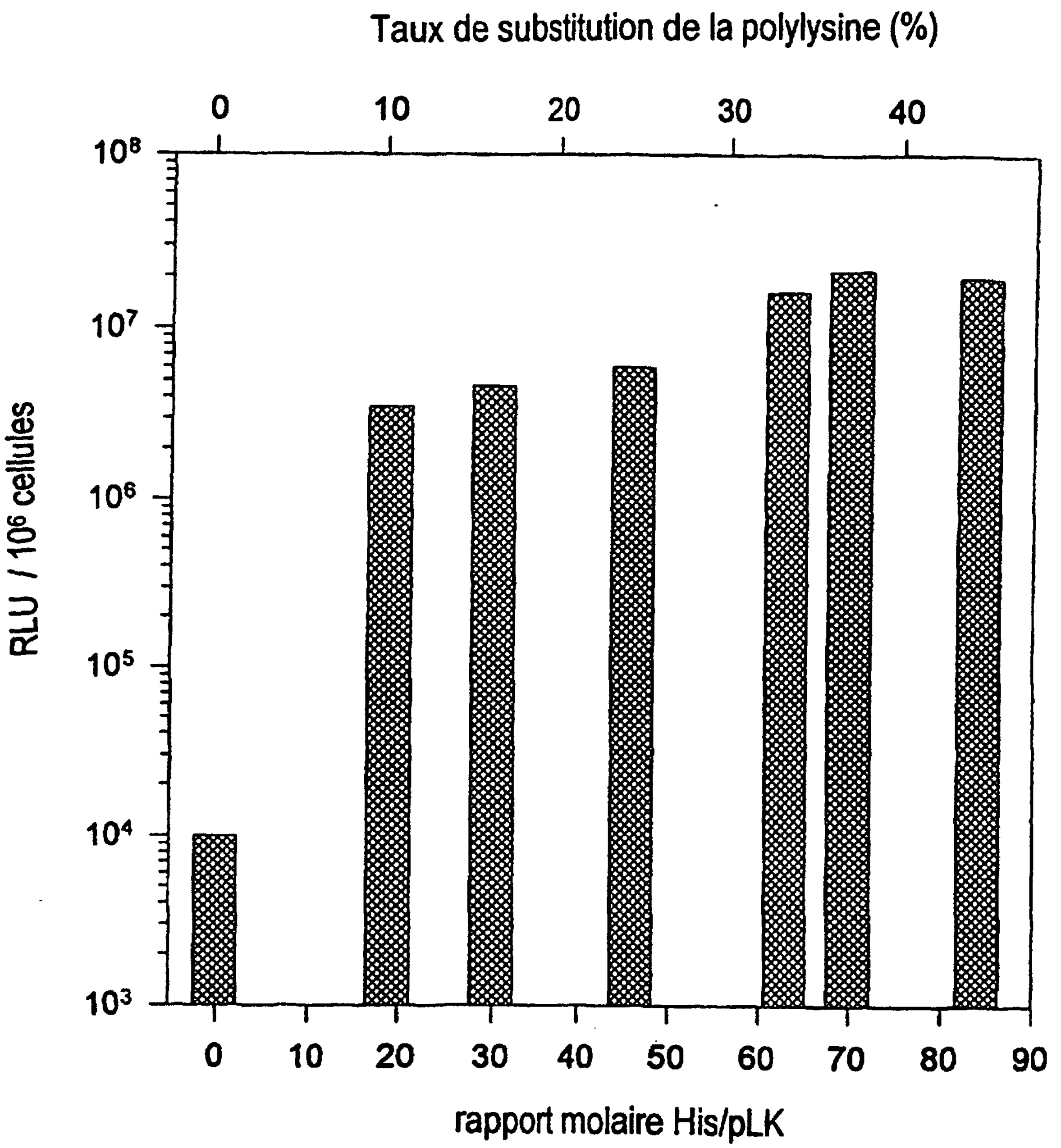
Taux de substitution de la polylysine (%)
0
10
20
30
40
$10^8$
$10^7$
$10^6$
$10^5$
$10^4$
$10^3$
RLU / $10^6$ cellules
0
10
20
30
40
50
60
70
80
90
rapport molaire His/pLK

Figure 5

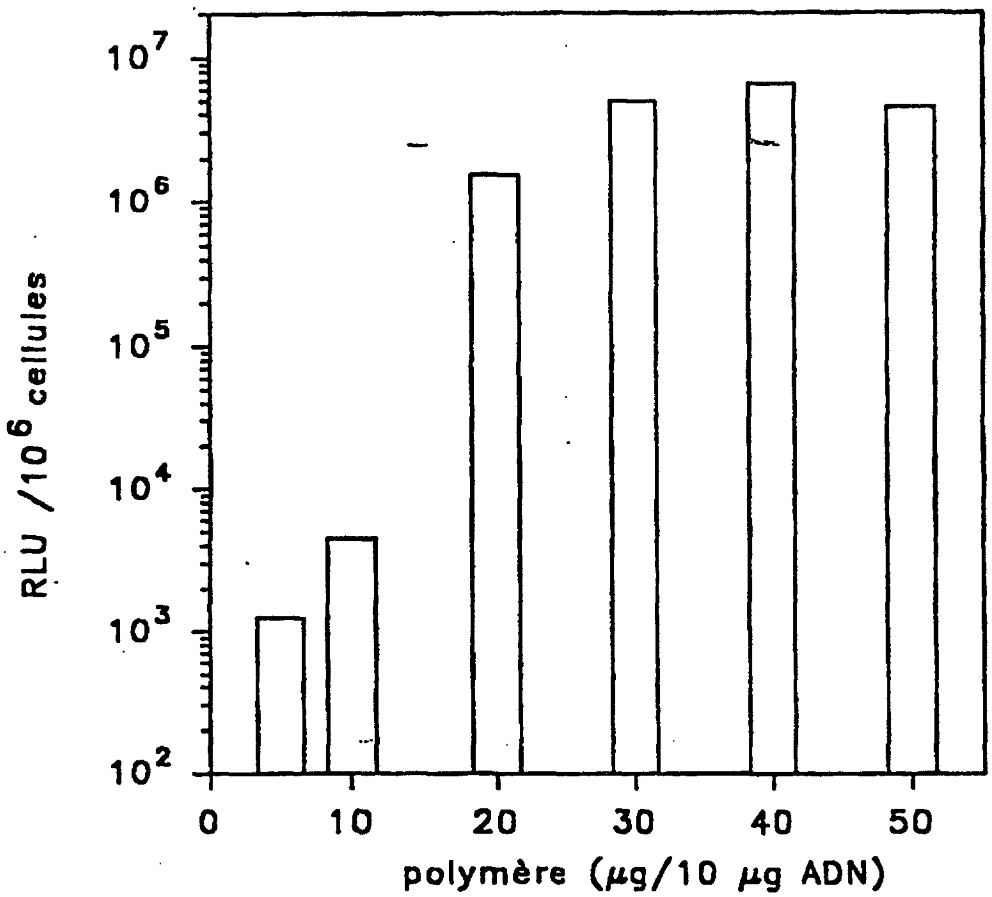
$10^7$
$10^6$
$10^5$
$10^4$
$10^3$
$10^2$
RLU /$10^6$ cellules
0
10
20
30
40
50
polymère (µg/10 µg ADN)

Figure 6

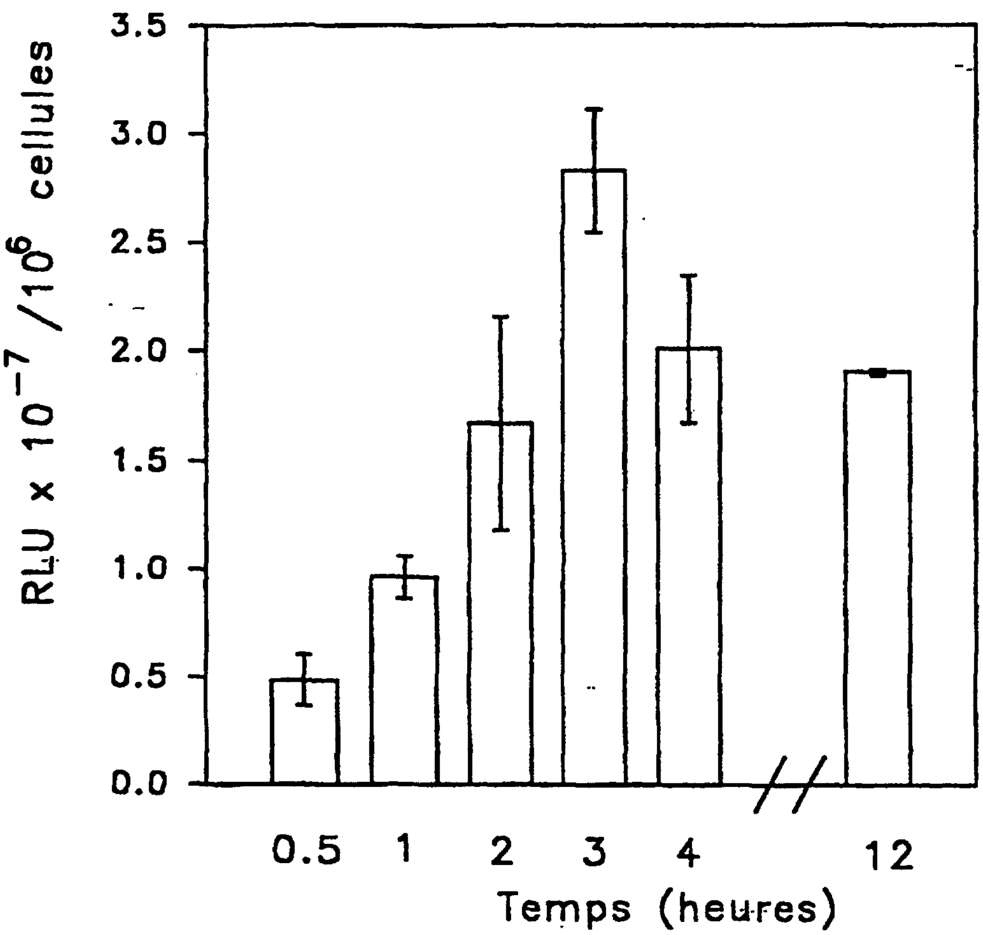
RLU x $10^{-7}$ /$10^6$ cellules
3.5
3.0
2.5
2.0
1.5
1.0
0.5
0.0
0.5
1
2
3
4
12
Temps (heures)

Figure 7

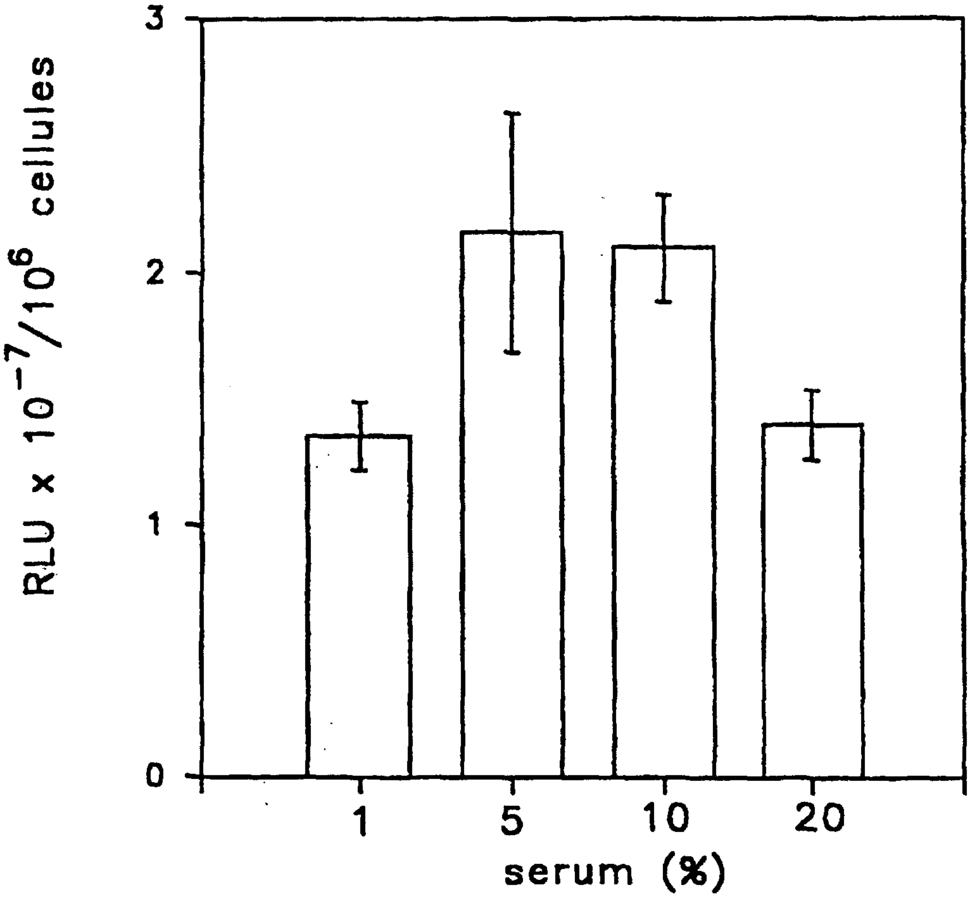


Figure 8

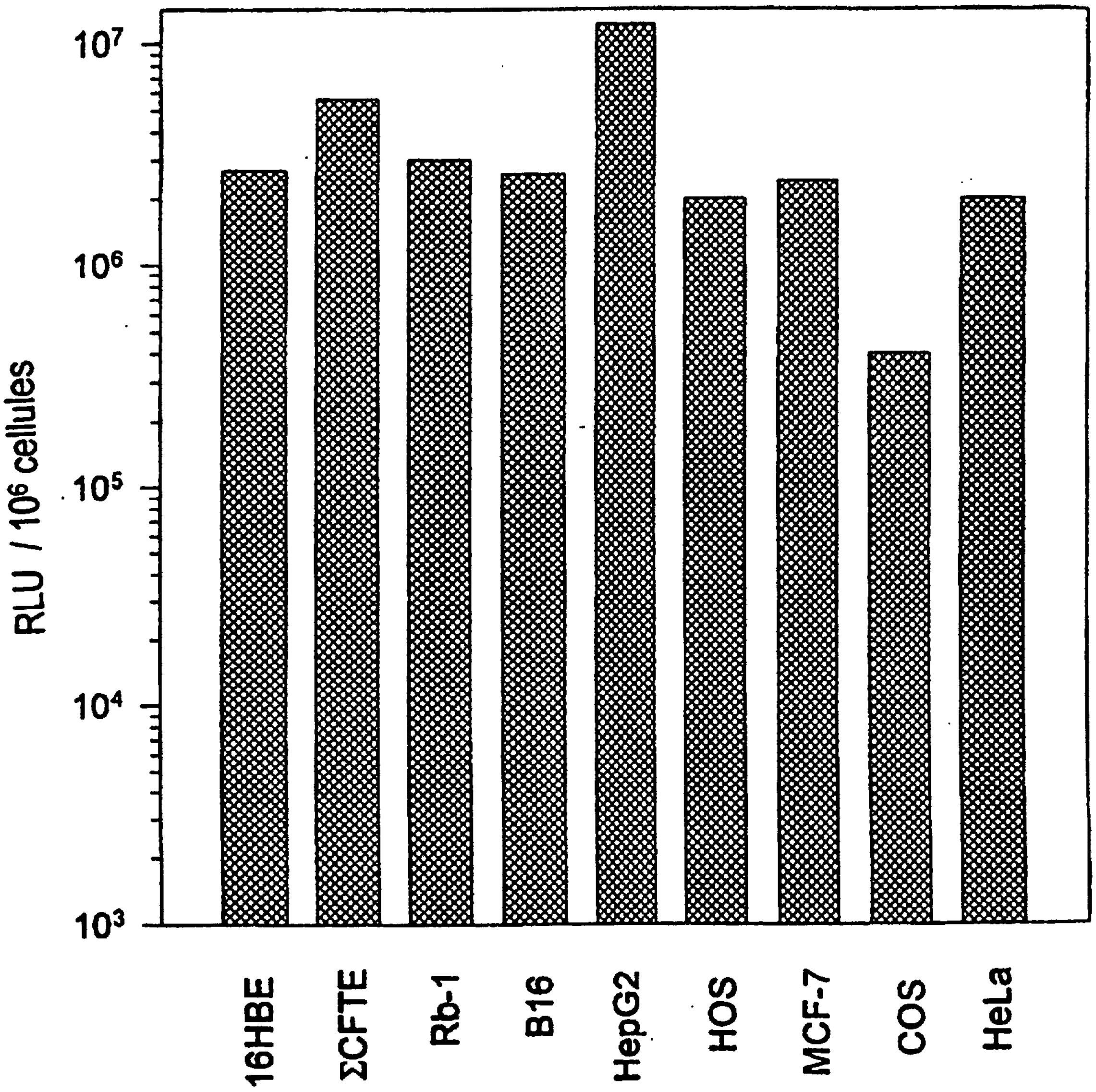

$10^7$
$10^6$
$10^5$
$10^4$
$10^3$
RLU / $10^6$ cellules
16HBE
ΣCFTE
Rb-1
B16
HepG2
HOS
MCF-7
COS
HeLa


Figure 9